# EUROPEAN PATENT SPECIFICATION

(11) **EP 1 141 298 B1**
(45) Date of publication and mention of the grant of the patent: **05.07.2006**
(21) Application number: 99966182.0
(22) Date of filing: 14.12.1999
(51) Int. Cl.: C12N 15/25, C12N 15/12, C07K 14/545, C07K 14/705, C07K 16/24, C12N 5/10, C12Q 1/68

(54) **IL-1 ZETA, IL-1 ZETA SPLICE VARIANTS AND XREC2 DNAS AND POLYPEPTIDES**
IL-1ZETA, IL-1ZETA SPLEISSVARIANTEN, UND XREC2 DNAS UND POLYPEPTIDE
POLYPEPTIDES ET ADN DE IL-1 ZETA, D'ALLELES D'EPISSAGE DE IL-1 ZETA, ET DE XREC2

(30) Priority: 14.12.1998 US 112163 P; 10.11.1999 US 164675 P
(43) Date of publication of application: 10.10.2001
(60) Divisional application: 05025531.4
(73) Proprietor: IMMUNEX CORPORATION, Seattle Washington 98101 (US)
(72) Inventor: SIMS, John, E., Seattle, WA 98105 (US); SMITH, Dirk, E., Bainbridge Island, WA 98110 (US); BORN, Teresa, L., Kenmore, WA 98028 (US)
(74) Representative: Lee, Nicholas John
(86) International application number: PCT/US1999/029549
(87) International publication number: WO 2000/036108

(56) References cited:
- WO-A-99/06426
- WO-A-99/06428
- "ou40f01.x1 Soares_NFL_T_GBC_S1 Homo sapiens cDNA clone IMAGE:1628761 3'" EMBL DATABASE ACCESSION NUMBER AI014548, 18 June 1998 (1998-06-18), XP002135000 cited in the application
- DINARELLO C A: "BIOLOGIC BASIS FOR INTERLEUKIN-1 IN DISEASE" BLOOD, vol. 87, no. 6, 15 March 1996 (1996-03-15), pages 2095-2147, XP000574933 ISSN: 0006-4971
- SMITH DE. ET AL.: "Four new members expand the interleukin-1 superfamily." J BIOL CHEM 275(2):1169-75, 14 January 2000 (2000-01-14), XP002135001

## Description

### BACKGROUND OF THE INVENTION

### Field of the Invention

The invention is directed to novel, purified and isolated IL-1 zeta, IL-1 zeta splice variants and fragments thereof, the nucleic acids encoding such polypeptides, processes for production of recombinant forms of such polypeptides, antibodies generated against these polypeptides, fragmented peptides derived from these polypeptides, and uses thereof.

### Description of Related Art

Interleukin-1 (IL-1) is a member of a large group of cytokines whose primary function is to mediate immune and inflammatory responses. There are five known IL-1 family members which include IL-1 alpha (IL-1α), IL-1 beta (IL-1β), IL-1 receptor antagonist (IL-1ra), IL-1 delta (IL-1δ-as disclosed in WO99/35268), and IL-18 (previously known as IGIF and sometimes IL-1 gamma). IL-1 that is secreted by macrophages is actually a mixture of mostly IL-1β and some IL-1α (Abbas et al., 1994). IL-1α and IL-1β, which are first produced as 33 kD precursors that lack a signal sequence, are further processed by proteolytic cleavage to produce secreted active forms, each about 17 kD. Additionally, the 33 kD precursor of IL-1α is also active. Both forms of IL-1 are the products of two different genes located on chromosome 2. Although the two forms are less than 30 percent homologous to each other, they both bind to the same receptors and have similar activities.

The IL-1 family of ligands binds to a common receptor composed of a ligand binding chain, the type I IL-1 receptor (IL-1RI), and a required signaling component, the IL-1R accessory protein (AcP) (Sims et al. 1988; Greenfeder et al. 1995; Cullinan et al. 1998). A type II IL-1 receptor (IL-1RII) binds and sequesters the agonist IL-1 (especially IL-1β) without inducing any signaling response of its own (McMahan et al. 1991; Sims et al. 1993; Colotta et al. 1993; Colotta et al. 1994). IL-1 ligands can also bind to a soluble proteolytic fragment of IL-1RII (sIL-1RII) (Colotta et al., 1993).

IL-1ra, a biologically inactive form of IL-1, is structurally homologous to IL-1. IL-1ra is produced with a signal sequence which allows for efficient secretion into the extracellular region (Abbas et al., 1994). Additionally, IL-1ra binds to the type I IL-1 receptor but fails to bring about the subsequent interaction with AcP. Thus, IL-Ira blocks IL-1RI and prevents the action of the agonist IL-1 (Hannum et al. 1990; Eisenberg et al. 1990).

The major source of IL-1 is the activated macrophage or mononuclear phagocyte. Other cells that produce IL-1 include epithelial and endothelial cells (Abbas et al., 1994). IL-1 secretion from macrophages occurs after the macrophage encounters and ingests gram-negative bacteria. Such bacteria contain lipopolysaccharide (LPS) molecules, also known as endotoxin, in the bacterial cell wall. LPS molecules are the active components that stimulate macrophages to produce tumor necrosis factor (TNF) and IL-1. In this case, IL-1 is produced in response to LPS and TNF production. At low concentrations, LPS stimulates macrophages and activates B-cells and other host responses needed to eliminate the bacterial infection; however, at high concentrations, LPS can cause severe tissue damage, shock, and even death.

The biological functions of IL-1 include activating vascular endothelial cells and lymphocytes, local tissue destruction, and fever (Janeway et al., 1996). At low levels, IL-1 stimulates macrophages and vascular endothelial cells to produce IL-6, upregulates molecules on the surface of vascular endothelial cells to increase leukocyte adhesion, and indirectly activates inflammatory leukocytes by stimulating mononuclear phagocytes and other cells to produce certain chemokines that activate inflammatory leukocytes. Additionally, IL-1 is involved in other inflammatory responses such as induction of prostaglandins, nitric oxide synthetase, and metalloproteinases. These IL-1 functions are crucial during low level microbial infections. However, if the microbial infection escalates, IL-1 acts systemically by inducing fever, stimulating mononuclear phagocytes to produce IL-1 and IL-6, increasing the production of serum proteins from hepatocytes, and activating the coagulation system. Additionally, IL-1 does not cause hemorrhagic necrosis of tumors, suppress bone marrow stem cell division, and IL-1 is lethal to humans at high concentrations.

Given the important function of IL-1, there is a need to identify additional members of the IL-1 ligand family and the IL-1 receptor family. In addition, in view of the continuing interest in protein research and the immune system, the discovery, identification, and roles of new proteins and their inhibitors, are at the forefront of modem molecular biology and biochemistry. Despite the growing body of knowledge, there is still a need in the art to discover the identity and function of proteins involved in cellular and immune responses.

In another aspect, the identification of the primary structure, or sequence, of an unknown protein is the culmination of an arduous process of experimentation. In order to identify an unknown protein, the investigator can rely upon a comparison of the unknown protein to known peptides using a variety of techniques known to those skilled in the art. For instance, proteins are routinely analyzed using techniques such as electrophoresis, sedimentation, chromatography, sequencing and mass spectrometry.

In particular, the unique nature of the composition of a protein with regard to its specific amino acid constituents results in unique positioning of cleavage sites within the protein. Specific fragmentation of a protein by chemical or enzymatic cleavage results in a unique "peptide fingerprint" (D. W. Cleveland et al., *J*. *Biol. Chem.* 252:1102-1106, 1977; M. Brown et al., *J. Gen. Virol.* 50:309-316, 1980). Consequently, cleavage at specific sites results in reproducible fragmentation of a given protein into peptides of precise molecular weights. Furthermore, these peptides possess unique charge characteristics that determine the isoelectric pH of the peptide. These unique characteristics can be exploited using a variety of electrophoretic and other techniques (Brock et al., Biology of Microorganisms, pp. 76-77, Prentice Hall, 6th ed. 1991).

Fragmentation of proteins is further employed for amino acid composition analysis and protein sequencing (P. Matsudiara, *J Biol. Chem.* 262:10035-10038, 1987; C. Eckerskorn et al., *Electrophoresis* 9:830-838, 1988), particularly the production of fragments from proteins with a "blocked" N-terminus. In addition, fragmented proteins can be used for immunization, for affinity selection (R. A. Brown, U.S. Patent No. 5,151,412), for determination of modification sites (e.g. phosphorylation), for generation of active biological compounds (Brock et al., Biology of Microorganisms. pp. 300-301, Prentice Hall, 6th ed. 1991), and for differentiation of homologous proteins (M. Brown et al., *J Gen. Virol.* 50:309-316, 1980).

In addition, when a peptide fingerprint of an unknown protein is obtained, it can be compared to a database of known proteins to assist in the identification of the unknown protein using mass spectrometry (W.J. Henzel et al., *Proc. Natl. Acad Sci. USA* 90:5011-5015, 1993; D. Fenyo et al., *Electrophoresis* 19:998-1005, 1998). A variety of computer software programs to facilitate these comparisons are accessible via the Internet, such as Protein Prospector (Internet site: prospector.uscf.edu), MultiIdent (Internet site: www.expasy.ch/sprot/multiident.html), PeptideSearch (Internet site: www.mann.embl-heiedelberg.de...deSearch/FR_PeptideSearch Form.html), and ProFound (Internet site: www.chait-sgi.rockefeller.edu/cgi-bin/prot-id-frag.html). These programs allow the user to specify the cleavage agent and the molecular weights of the fragmented peptides within a designated tolerance. The programs compare these molecular weights to protein molecular weight information stored in databases to assist in determining the identity of the unknown protein. Accurate information concerning the number of fragmented peptides and the precise molecular weight of those peptides is required for accurate identification. Therefore, increasing the accuracy in determining the number of fragmented peptides and their molecular weight should result in enhanced likelihood of success in the identification of unknown proteins.

In addition, peptide digests of unknown proteins can be sequenced using tandem mass spectrometry (MS/MS) and the resulting sequence searched against databases (J.K. Eng et al., *J. Am. Soc. Mass Spec.* 5:976-989, 1994; M. Mann et al., *Anal. Chem.* 66:4390-4399, 1994; J.A. Taylor et al., *Rapid Comm. Mass Spec.* 11:1067-1075, 1997). Searching programs that can be used in this process exist on the Internet, such as Lutefisk 97 (Internet site: www.Isbc.com:70/Lutefisk97.html), and the Protein Prospector, Peptide Search and ProFound programs described above. Therefore, adding the sequence of a gene and its predicted protein sequence and peptide fragments to a sequence database can aid in the identification of unknown proteins using tandem mass spectrometry.

Thus, there also exists a need in the art for polypeptides suitable for use in peptide fragmentation studies, for use in molecular weight measurements, and for use in protein sequencing using tandem mass spectrometry.

### SUMMARY OF THE INVENTION

The present invention provides isolated nucleic acids and polypeptides encoded by the nucleic acids for an IL-1 family ligand termed "IL-1 zeta" and two splice variants of IL-1 zeta, termed TDZ.1 and TDZ.2. Thus, in one aspect, the invention is directed to an isolated polynucleotide comprising a nucleic acid molecule that encodes a polypeptide selected from the group consisting of
(a) a polypeptide comprising SEQ ID NO: 3;
(b) a polypeptide comprising SEQ ID NO: 8;
(c) a polypeptide comprising SEQ ID NO: 9;
(d) a polypeptide that is at least 95% identical to a polypeptide of a) or c), wherein the polypeptide binds an IL-1R family member; and
(e) a fragment of the polypeptide of SEQ ID NO: 3 whose N-terminal amino acid is selected from amino acids 21-51 and whose C-terminal amino acid is selected from amino acids 188-192 and which binds an IL-1R family member, wherein the fragment is not amino acids 24-192, 28-192 or 34-192 of SEQ ID NO: 3;
(f) a fragment of the polypeptide of SEQ ID NO: 8 whose N-terminal amino acid is selected from amino acids 41-61 and whose C-terminal amino acid is selected from amino acids 214-218 and which binds an IL-1 R family member,
   wherein the fragment is not amino acids 43-218, 54-218 or 60-218 of SEQ ID NO: 8; and
(g) a fragment of the polypeptide of SEQ ID NO: 9 whose N-terminal amino acid is selected from amino acids 21-41 and whose C-terminal amino acid is selected from amino acids 193-197 and which binds an IL-1R family member, wherein the fragment is not amino acids 33-197 or 39-197 of SEQ ID NO: 9.

The nucleic acid molecule may have the sequence of SEQ ID NO: 1 or SEQ ID NO: 6.

In another aspect, the invention is directed to an isolated polypeptide comprising a polypeptide selected from the group consisting of
(a) a polypeptide comprising SEQ ID NO: 3;
(b) a polypeptide comprising SEQ ID NO: 8;
(c) a polypeptide comprising SEQ ID NO: 9;
(d) a polypeptide that is at least 95% identical to a polypeptide of a)-c), wherein the polypeptide binds an IL-1R family member;
(e) a fragment of the polypeptide of SEQ ID NO: 3 whose N-terminal amino acid is selected from amino acids 21-51 and whose C-terminal amino acid is selected from amino acids 188-192 and which binds an IL-1R family member, wherein the fragment is not amino acids 24-192, 28-192 or 34-192 of SEQ ID NO: 3;
(f) a fragment of the polypeptide of SEQ ID NO: 8 whose N-terminal amino acid is selected from amino acids 41-61and whose C-terminal amino acid is selected from amino acids 214-218 and which binds an IL-1R family member, wherein the fragment is not amino acids 43-218, 54-218 or 60-218 of SEQ ID NO: 8; and
(g) a fragment of the polypeptide of SEQ ID NO: 9 whose N-terminal amino acid is selected from amino acids 21-41 and whose C-terminal amino acid is selected from amino acids 193-197 and which binds an IL-1 R family member, wherein the fragment is not amino acids 33-197 or 39-197 of SEQ ID NO: 9.

In the polynucleotide and the polypeptide of the invention, wherein the N-terminal amino acid of the fragment of the polypeptide of SEQ ID NO: 8 may be amino acid 52 and the N-terminal amino acid of the fragment of the polypeptide of SEQ ID NO: 9 may be amino acid 31

The invention also provides an oligomeric polypeptide comprising a polypeptide of the invention.

Both single-stranded and double-stranded RNA and DNA nucleic acid molecules are encompassed by the invention. The invention also encompasses an expression vector comprising a polynucleotide of the invention and a host cell transformed or transfected with such a vector.

The nucleic acids noted above can be used to identify nucleic acids encoding proteins having activities associated with IL-1 family ligands and receptors. Thus, IL-1 zeta nucleic acid molecules can be used to identify the IL-1 zeta receptor.

In addition, these nucleic acids can be used to identify the human chromosomes with which the nucleic acids are associated. Thus, the IL-1 zeta, TDZ.1 and TDZ.2 nucleic acids can be used to identify human chromosome 2. Accordingly, these nucleic acids can also be used to map genes on human chromosome 2; to identify genes associated with certain diseases, syndromes, or other human conditions associated with human chromosome 2, and to study cell signal transduction and the immune system.

Sense or antisense oligonucleotides from the nucleic acids of SEQ ID NOs:1, 2, 5, 6 and 7 can be used to inhibit the expression of the respective polynucleotide encoded by the nucleic acids.

The invention further encompasses a method for preparing a polypeptide, comprising culturing a host cell under conditions promoting expression of the polypeptide. The polypeptide may then be recovered from the culture medium. Especially, the expression of these polypeptides in bacteria, yeast, plant, insect, and animal cells is encompassed by the invention.

In general, the polypeptides of the invention can be used to study cellular processes such as immune regulation, cell proliferation, cell death, cell migration, cell-to-cell interaction, and inflammatory responses. In addition, these polypeptides can be used to identify proteins associated with IL-1 zeta, TDZ.1 and TDZ.2.

In addition, these polypeptides can be utilised in assays to screen for potential inhibitors of activity associated with polypeptide counter-structure molecules, and as therapeutic agents for the treatment of diseases mediated by polypeptide counter-structure molecules. Further, these polypeptides can be used in the design of inhibitors (e.g., engineered receptors that act as inhibitors) thereof.

The IL-1 zeta nucleic acid sequences, predicted amino acid sequences of the polypeptide or fragments thereof, or a combination of the predicted amino acid sequences of the polypeptide and fragments thereof can be used in searching an electronic database to aid in the identification of sample nucleic acids and/or proteins. The polypeptides disclosed herein can be used as controls for establishing the extent of protein fragmentation.

Isolated polyclonal or monoclonal antibodies that bind to these polypeptides are also encompassed by the invention. These antibodies can be used to aid in purifying the polypeptides of the invention.

### BRIEF DESCRIPTION OF THE FIGURES

Figure 1 diagrams the genomic structure of the IL-1 zeta locus.
Figure 2 represents a molecular model showing the secondary structure of IL-1 zeta, in which β-strands are shown in yellow, with their direction indicated by the arrowhead; β-turns are shown in blue; and coils are shown in green. The IL-1 zeta structure is presented in two different views.

### DETAILED DESCRIPTION OF THE INVENTION

The nucleic acid molecules encompassed in the invention include the following nucleotide sequences:
**Name: IL-1-zeta**
**Name: Xrec2 (not encompassed in the invention)**
**Name: TDZ.1**
**Name: TDZ.2**
**Name: TDZ.3 (not encompassed in the invention)**

The amino acid sequences of the polypeptides encoded by the nucleotide sequence of the invention include:
**Name: IL-1 zeta (polypeptide)**
**Name: Xrec2 (polypeptide) (not encompassed in the invention)**
**TDZ.1 (polypeptide)**
**Name: TDZ.2 (polypeptide)**
Name: TDZ.3 (polypeptide) (not encompassed in the invention)

The discovery of the IL-I zeta, the IL-1 zeta splice variants (TDZ.1, TDZ.2, and TDZ.3) and the Xrec2 nucleic acids enables the construction of expression vectors comprising nucleic acid sequences encoding the respective polypeptides and host cells transfected or transformed with the expression vectors. The isolation and purification of biologically active IL-1 zeta, the IL-1 zeta splice variants, and Xrec2 polypeptides and fragments thereof is also enabled. The nucleic acids or oligonucleotides thereof can be used as probes to identify nucleic acid encoding proteins having associated activities. Thus, IL-1 zeta and the IL-1 zeta splice variants can be used to identify activities associated with IL-1 family ligands and Xrec2 can be used to identify activities associated with IL-1 family receptors. In addition, the nucleic acids or oligonucleotides thereof of IL-1 zeta TDZ.1, TDZ.2, and TDZ.3 can be used to identify human chromosomes 2, while those of Xrec2 can be used to identify human chromosome X. Similarly, these nucleic acids or oligonucleotides thereof can be used to map genes on human chromosomes 2 and X. respectively, and to identify genes associated with certain diseases, syndromes or other human conditions associated with human chromosomes 2 and X. Thus, the nucleic acids or oligonucleotides thereof of IL-1 zeta, TDZ.1, TDZ.2, and TDZ.3 can be used to identify glaucoma, ectodermal dysplasia, insulin-dependent diabetes mellitus, wrinkly skin syndrome, T-cell leukemia/lymphoma, and tibial muscular dystrophy, while the nucleic acids or oligonucleotides thereof of Xrec2 can be used to identify retinoschisis, lissencephaly, subcortical laminalheteropia, mental retardation, cowchock syndrome, bazex syndrome, hypertrichosis, lymphoproliferative syndrome, immunodeficiency, Langer mesomelic dysplasia, and leukemia. Finally, single-stranded sense or antisense oligonucleotides from these nucleic acids can be used to inhibit expression of polynucleotides encoded by the IL-1 zeta and Xrec2 genes, respectively.

Further, the IL-1 zeta, TDZ.1, TDZ.2, TDZ.3 and Xrec2 polypeptides and soluble fragments thereof can be used to activate and/or inhibit the activation of vascular endothelial cells and lymphocytes, induce and/or inhibit the induction of local tissue destruction and fever (Janeway et al., 1996), inhibit and/or stimulate macrophages and vascular endothelial cells to produce IL-6, induce and/or inhibit the induction of prostaglandins, nitric oxide synthetase, and metalloproteinases, and upregulate and/or inhibit the upregulation of molecules on the surface of vascular endothelial cells. In addition these polypeptides and fragmented peptides can also be used to induce and/or inhibit the induction of inflammatory mediators such as transcription factors NF-κB and AP-1, MAP kinases JNK and p38, COX-2, iNOS, and all of the activities stimulated by these molecules.

In addition, these polypeptides and fragmented peptides can be used as controls for peptide fragmentation. Finally, these polypeptides and fragments thereof can be used to generate antibodies, and the invention includes the use of such antibodies to purify IL-1 zeta and Xrec2 polypeptides.

### NUCLEIC ACID MOLECULES

In a particular embodiment, the invention relates to certain isolated nucleotide sequences that are free from contaminating endogenous material. A "nucleotide sequence" refers to a polynucleotide molecule in the form of a separate fragment or as a component of a larger nucleic acid construct. The nucleic acid molecule has been derived from DNA or RNA isolated at least once in substantially pure form and in a quantity or concentration enabling identification, manipulation, and recovery of its component nucleotide sequences by standard biochemical methods (such as those outlined in Sambrook et al., Molecular Cloning: A Laboratory Manual, 2nd ed., Cold Spring Harbor Laboratory, Cold Spring Harbor, NY, 1989). Such sequences are preferably provided and/or constructed in the form of an open reading frame uninterrupted by internal non-translated sequences, or introns, that are typically present in eukaryotic genes. Sequences of non-translated DNA can be present 5' or 3' from an open reading frame, where the same do not interfere with manipulation or expression of the coding region.

Nucleic acid molecules of the invention include DNA in both single-stranded and double=stranded form, as well as the RNA complement thereof. DNA includes, for example, cDNA, genomic DNA, chemically synthesized DNA, DNA amplified by PCR, and combinations thereof. Genomic DNA may be isolated by conventional techniques, *e.g.,* using the cDNA of SEQ ID NOs:1, 2,5, 6, 7or a suitable fragment thereof, as a probe.

The DNA molecules of the invention include full length genes as well as polynucleotides and fragments thereof. The full length gene may include the N-terminal signal peptide. Other embodiments include DNA encoding a soluble form, e.g., encoding the extracellular domain of the protein, either with or without the signal peptide.

The nucleic acids of the invention are preferentially derived from human sources, but the invention includes those derived from non-human species, as well.

### Preferred Sequences

The particularly preferred nucleic acid molecules of the invention are those shown in SEQ ID NOs: 1 and 6 for IL-1 zeta and TDZ.2, respectively. cDNA clones having the nucleic acid sequence of SEQ ID NOs: 1 and 2 were isolated as described in Example 1. The sequences of the amino acids of IL-1 zeta and Xrec2 encoded by the DNAs of SEQ ID NOs:1 and 2 are shown in SEQ ID NOs:3 and 4, respectively. cDNA clones having the nucleic acid sequence of SEQ ID NOs:5, 6, and 7 were isolated as described in Example 8. The sequences of the amino acids of TDZ.1, TDZ.2, and TDZ.3 encoded by the DNAs of SEQ ID NOs:5, 6, and 7 are shown in SEQ ID NOs:8, 9, and 10, respectively.

SEQ ID NOs:1-4 identify the IL-1 zeta of SEQ ID NO:3 as a member of the IL-1 family and the Xrec2 of SEQ ID NO:4 as a member of the IL-1 receptor family. The homologies on which this is based are set forth at Table I.

**TABLE I**

| **Protein** | **Source** | **Percent identity to IL-1 zeta** |
|---|---|---|
| IL-1 alpha | Human | 21% |
| IL-1 beta | Human | 24% |
| IL-1 delta | Human | 34% |
| IL-18 | Human | 21 % |
| IL-1ra | Human | 29% |

| **Protein** | **Source** | **Percent identity to Xree2** |
|---|---|---|
| TIGIRR (IL-1 R family member) | Human | 63% |
| TIGIRR (IL-1R family member) | Murine | 61% |
| SIGIRR | Human | 22% |
| IL-1R-AcP | Human | 35% |
| IL-1R-AcPL | Human | 26% |
| IL-1R | Human | 29% |
| RP 1 | Human | 31% |
| RP2 | Human | 28% |
| ST2 | Human | 26% |

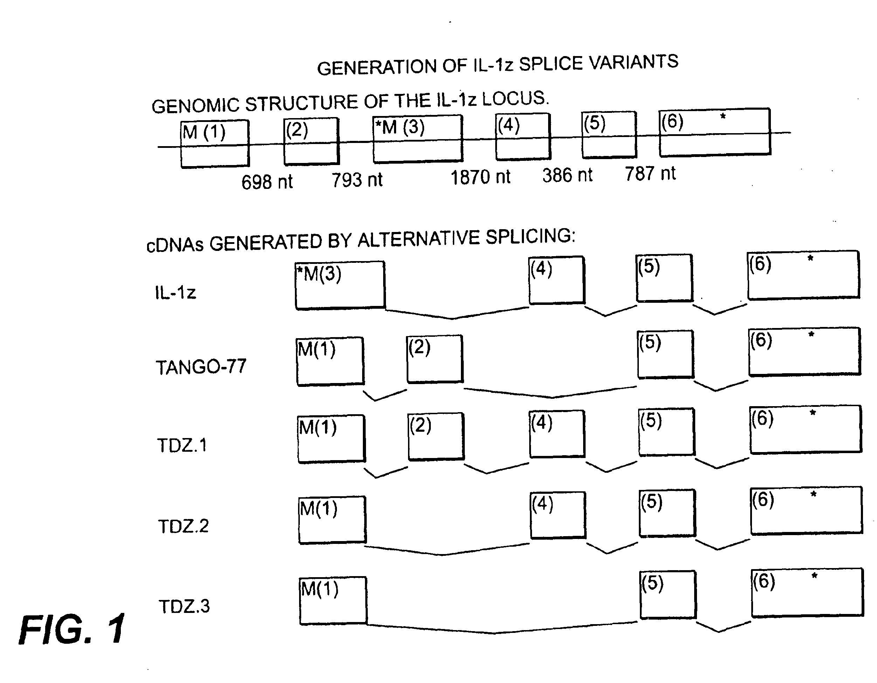

The IL-1 zeta splice variants were discovered in a stretch of genomic DNA sequence (X22304.gbn). This genomic sequence also contains the different IL-1 zeta exons and another splice variant known as Tango-77 (WO 99/06426). Comparing the cDNA sequences of the cloned IL-1 zeta, TDZ.1, TDZ.2, TDZ.3 and Tango-77 with the genomic sequence provides insight into the generation of the splicing events. Figure 1 shows the genomic structure of the IL-1 zeta locus and the cDNAs generated by alternative splicing. The numbered boxes indicate individual exons 1-6 and the approximate size of the intervening introns is indicated at the top. The asterisk (*) indicates the presence of a stop codon, at the end of the coding sequence (exon 6) or as an in-frame stop codon (exon 3). "M" indicates a potential initiating methionine originating either from exon 1 or exon 3. Tango-77 is the cDNA structure disclosed in WO 99/06426. A significant feature of IL-1 zeta and its splice variants is the presence or the absence of exon 4. Exon 4 is present in IL-1 zeta, TDZ.1 and TDZ.2 but not in Tango-77 or TDZ.3. The amino acid sequence encoded by exon 4 aligns well with the amino acid sequences of other IL-1 family members in the first few beta strands of the mature peptides. By contrast, the amino acid sequences encoded by exons 1 and 2 of Tango-77 and exon 1 of TDZ.3 cDNAs, which replace rather than supplement exon 4 of IL-1 zeta, TDZ.1 and TDZ.2, do not align well with other IL-1 family members in this same region. IL-1 zeta, Tango-77, TDZ.1, TDZ.2, and TDZ.3 all align well with amino acid sequences of other IL-1 family members in the C-terminal 2/3 of the mature peptide (the region encoded by exons 5 and 6 which are common to all of these splice isoforms). Thus, the "mature peptides" encoded by IL 1 zeta, TDZ.1 and TDZ.2 DNAs are likely to represent functional IL-1 like molecules. This contrasts with the polypeptides encoded by Tango-77 or TDZ.3 DNAs which are less likely to represent a functional IL-1 like molecule.

It is probable that all of the splice isoforms, except TDZ.3, encode proforms of an IL-1 like cytokine, since in the N-terminal direction the cDNAs extend well beyond the N-terminus of mature IL-1s. This observation predicts that IL-1 zeta, TDZ.1 and TDZ.2 encode the same mature peptide. In connection with this observation it is the prodomains (as well as 5' UTRs) that differ between IL-1 zeta, TDZ.1 and TDZ.2.

Table III, which details the tissue distribution of IL-1 zeta, TDZ.1, TDZ.2, TDZ.3 and Tango-77, shows that the expression of Tango-77 is more widespread than that of IL-1 zeta. Table III also shows that the TDZ.1 expression is comparable, and almost entirely overlapping, with that of Tango-77. The tissue distribution data combined with the alignment information of Figure 1 show that TDZ.1 is the only member of the splice variants that aligns well with other IL-1 family members, and is widespread in its expression. These observations suggest that TDZ.1 may be the most significant of the splice variants in terms of relevance to biological responses.

### Additional Sequences

Due to the known degeneracy of the genetic code, wherein more than one codon can encode the same amino acid, a DNA sequence can vary from that shown in SEQ ID NOs: 1, 2, 5, 6, and 7 and still encode a polypeptide having the amino acid sequence of SEQ ID NOs:3, 4, 8, 9, and 10, respectively. Such variant DNA sequences can result from inadvertent mutations (e.g., occurring during PCR amplification), or can be the product of deliberate mutagenesis of a native sequence.

The invention provides isolated DNA sequences encoding polypeptides of the invention, selected from: (a) DNA comprising the nucleotide sequence of SEQ ID NOs:1, 5, and 6 and (b) DNA encoding the polypeptides of SEQ ID NOs:3, 8, and 9. Of course, polypeptides encoded by such DNA sequences are encompassed by the invention.

As used herein, conditions of moderate stringency can be readily determined by those having ordinary skill in the art based on, for example, the length of the DNA. The basic conditions are set forth by Sambrook et al., Molecular Cloning: A Laboratory Manual. 2nd ed. Vol. 1, pp. 1.101-104, Cold Spring Harbor Laboratory Press, 1989, and include use of a prewashing solution for the nitrocellulose filters 5X SSC, 0.5% SDS, 1.0 mM EDTA (pH 8.0), hybridization conditions of about 50% formamide, 6X SSC at about 42°C (or other similar hybridization solution, such as Stark's solution, in about 50% formamide at about 42°C), and washing conditions of about 60°C, 0.5X SSC, 0.1% SDS. Conditions of high stringency can also be readily determined by the skilled artisan based on, for example, the length of the DNA. Generally, such conditions are defined as hybridization conditions as above, and with washing at approximately 68°C, 0.2X SSC, 0.1% SDS. The skilled artisan will recognize that the temperature and wash solution salt concentration can be adjusted as necessary according to factors such as the length of the probe.

Also included as an embodiment of the invention is DNA encoding certain polypeptide fragments and polypeptides comprising inactivated N-glycosylation site(s), inactivated protease processing site(s), or conservative amino acid substitution(s), as described below.

Nucleic acid molecules may comprise nucleotide sequences that are at least 80% identical, at least 90% identical, at least 95% identical, at least 98% identical, at least 99% identical, or at least 99.9% identical to a native sequence.

The percent identity may be determined by visual inspection and mathematical calculation. Alternatively, the percent identity of two nucleic acid sequences can be determined by comparing sequence information using the GAP computer program, version 6.0 described by Devereux et al., *Nucl. Acids Res.* 12:387, 1984, and available from the University of Wisconsin Genetics Computer Group (UWGCG). The preferred default parameters for the GAP program include: (1) a unary comparison matrix (containing a value of 1 for identities and 0 for non-identities) for nucleotides, and the weighted comparison matrix of Gribskov and Burgess, *Nucl. Acids Res.* 14:6745, 1986, as described by Schwartz and Dayhoff, eds., Atlas of Protein Sequence and Structure, pp. 353-358, National Biomedical Research Foundation, 1979; (2) a penalty of 3.0 for each gap and an additional 0.10 penalty for each symbol in each gap; and (3) no penalty for end gaps. Other programs used by one skilled in the art of sequence comparison may also be used.

The invention provides isolated nucleic acids useful in the production of polypeptides. Such polypeptides may be prepared by any of a number of conventional techniques. A DNA sequence encoding a polypeptide of the invention, or desired fragment thereof may be subcloned into an expression vector for production of the polypeptide or fragment. The DNA sequence advantageously is fused to a sequence encoding a suitable leader or signal peptide. Alternatively, the desired fragment may be chemically synthesized using known techniques. DNA fragments also may be produced by restriction endonuclease digestion of a full length cloned DNA sequence, and isolated by electrophoresis on agarose gels. If necessary, oligonucleotides that reconstruct the 5' or 3' terminus to a desired point may be ligated to a DNA fragment generated by restriction enzyme digestion. Such oligonucleotides may additionally contain a restriction endonuclease cleavage site upstream of the desired coding sequence, and position an initiation codon (ATG) at the N-terminus of the coding sequence.

The well-known polymerase chain reaction (PCR) procedure also may be employed to isolate and amplify a DNA sequence encoding a desired protein fragment. Oligonucleotides that define the desired termini of the DNA fragment are employed as 5' and 3' primers. The oligonucleotides may additionally contain recognition sites for restriction endonucleases, to facilitate insertion of the amplified DNA fragment into an expression vector. PCR techniques are described in Saiki et al., *Science,* 239:487, 1988; Wu et al., eds., Recombinant DNA Methodology, pp. 189-196, Academic Press, Inc., San Diego, 1989; and Innis et al., eds., PCR Protocols: A Guide to Methods and Applications, Academic Press, Inc., 1990.

### POLYPEPTIDES AND FRAGMENTS THEREOF

The invention encompasses polypeptides and fragments thereof in various forms, including those that are naturally occurring or produced through various techniques such as procedures involving recombinant DNA technology. Such forms include, but are not limited to, derivatives, variants, and oligomers, as well as fusion proteins or fragments thereof.

The polypeptides of the invention include full length proteins encoded by the nucleic acid sequences of the invention set forth above. Polypeptides of IL-1 zeta, TDZ.1, TDZ.2,TDZ.3 and Xrec2 comprise the amino acid sequence of SEQ ID NOs:3, 4, 8, 9, and 10 respectively. For IL-1 zeta, TDZ.1, TDZ.2, and TDZ-3,the N-terminus does not encode a classical signal peptide but the extra length relative to the mature form of other IL-1 family members suggests that the N-terminus may act as a prodomain. A predicted cleavage site is the point where the conserved structural portion of the protein begins. Structural modeling data support this assumption. For IL-1 zeta, TDZ.1, and TDZ.2 the site is somewhere near the N-terminal end of the amino acid sequence encoded by exon 4. Thus, the polypeptide of IL-1 zeta, as set forth in SEQ ID NO:3, includes a putative prodomain that extends from amino acids 1 to x, where x is an integer from 20 to 50. Similarly, TDZ.1 of SEQ ID NO: 8 includes a putative prodomain that extends from amino acids 1 to x' where x' is an integer from 40-60 and most preferably x' is about 52. TDZ.2 of SEQ ID NO:9 includes a putative prodomain that extends from amino acids 1 to x", where x" is an integer from 20-40 and most preferable x" is 31.

Unlike IL-1 zeta and its splice variants, the polypeptide of Xrec2, as set forth in SEQ ID N0:4, includes an N-terminal hydrophobic region that functions as a signal peptide, followed by an extracellular domain comprising amino acids 19 to 359, a transmembrane region comprising amino acids 360 through 378, and a C-terminal cytoplasmic domain comprising amino acids 379 to 696. Computer analysis predicts that the signal peptide corresponds to residues 1 to 19 of SEQ ID NO:4 (although the next most likely computer-predicted signal peptide cleavage sites, in descending order, occur after amino acids 20 and 16 of SEQ ID NO:4). Cleavage of the signal peptide thus would yield a mature protein comprising amino acids 19 through 696 of SEQ ID NO:4.

The skilled artisan will recognize that the above-described boundaries of such regions of the polypeptide are approximate. To illustrate, the boundaries of the transmembrane region (which may be predicted by using computer programs available for that purpose) may differ from those described above.

The polypeptides of the invention may be membrane bound or they may be secreted and, thus, soluble. Soluble polypeptides are capable of being secreted from the cells in which they are expressed. In general, soluble polypeptides may be identified (and distinguished from non-soluble membrane-bound counterparts) by separating intact cells which express the desired polypeptide from the culture medium, e.g., by centrifugation, and assaying the medium (supernatant) for the presence of the desired polypeptide. The presence of polypeptide in the medium indicates that the polypeptide was secreted from the cells and thus is a soluble form of the protein.

In one embodiment, the soluble polypeptides and fragments thereof comprise all or part of the extracellular domain, but lack the transmembrane region that would cause retention of the polypeptide on a cell membrane. A soluble polypeptide may include the cytoplasmic domain, or a portion thereof, as long as the polypeptide is secreted from the cell in which it is produced.

In general, the use of soluble forms is advantageous for certain applications. Purification of the polypeptides from recombinant host cells is facilitated, since the soluble polypeptides are secreted from the cells. Further, soluble polypeptides are generally more suitable for intravenous administration.

The invention also provides certain polypeptides and fragments of the extracellular domain that retain a desired biological activity. Particular embodiments are directed to polypeptide fragments of SEQ ID NOs:3, 8, and 9 that retain the ability to bind the native cognates, substrates, or counter-structure ("binding partner). Such a fragment may be a soluble polypeptide, as described above. In another embodiment, the polypeptides and fragments advantageously include regions that are conserved in the IL-1 ligand and IL-1 receptor family as described above.

Polypeptide fragments may comprise at least 20, or at least 30, contiguous amino acids of the sequences of SEQ ID NOs:3, 4, 8, 9, and 10. Fragments derived from the cytoplasmic domain of Xrec2 of SEQ ID NO:4 find use in the studies of signal transduction, and in regulating cellular processes associated with transduction of biological signals. Polypeptide fragments also may be employed as immunogens, in generating antibodies.

### Variants

Naturally occurring variants as well as derived variants of the polypeptides and fragments are provided herein.

Variants may exhibit amino acid sequences that are at least 80% identical, at least 90% identical, at least 95% identical, at least 98% identical, at least 99% identical, or at least 99.9% identical to the preferred polypeptide or fragment thereof. The present invention provides an isolated polypeptide that is at least 95% identical to a polypeptide comprising SEQ ID NO:3, 8 or 9 wherein the polypeptide binds an IL-1R family member, and an isolated polynucleotide comprising a nucleic acid molecule encoding such a polypeptide. Percent identity may be determined by visual inspection and mathematical calculation. Alternatively, the percent identity of two protein sequences can be determined by comparing sequence information using the GAP computer program, based on the algorithm of Needleman and Wunsch *(J. Mol. Bio.* 48:443, 1970) and available from the University of Wisconsin Genetics Computer Group (UWGCG). The preferred default parameters for the GAP program include: (1) a scoring matrix, blosum62, as described by Henikoff et al., *Proc. Natl. Acad. Sci. USA,* 89:10915, 1992; (2) a gap weight of 12; (3) a gap length weight of 4; and (4) no penalty for end gaps. Other programs used by one skilled in the art of sequence comparison may also be used.

The variants of the invention include, for example, those that result from alternate mRNA splicing events or from proteolytic cleavage. Alternate splicing of mRNA may, for example, yield a truncated but biologically active protein, such as a naturally occurring soluble form of the protein. Variations attributable to proteolysis include, for example, differences in the N- or C-termini upon expression in different types of host cells, due to proteolytic removal of one or more terminal amino acids from the protein (generally from 1-5 terminal amino acids). Proteins in which differences in amino acid sequence are attributable to genetic polymorphism (allelic variation among individuals producing the protein) are also contemplated herein.

Additional variants within the scope of the invention include polypeptides that may be modified to create derivatives thereof by forming covalent or aggregative conjugates with other chemical moieties, such as glycosyl groups, lipids, phosphate, acetyl groups and the like. Covalent derivatives may be prepared by linking the chemical moieties to functional groups on amino acid side chains or at the N-terminus or C-terminus of a polypeptide. Conjugates comprising diagnostic (detectable) or therapeutic agents attached thereto are contemplated herein, as discussed in more detail below.

Other derivatives include covalent or aggregative conjugates of the polypeptides with other proteins or polypeptides, such as by synthesis in recombinant culture as N-terminal or C-terminal fusions. Examples of fusion proteins are discussed below in connection with oligomers. Further, fusion proteins can comprise peptides added to facilitate purification and identification. Such peptides include, for example, poly-His or the antigenic identification peptides described in U.S. Patent No. 5,011,912 and in Hopp et al., *Bio*/*Technology,* 6:1204, 1988. One such peptide is the FLAG® peptide, Asp-Tyr-Lys-Asp-Asp-Asp-Asp-Lys (SEQ ID NO:11), which is highly antigenic and provides an epitope reversibly bound by a specific monoclonal antibody, enabling rapid assay and facile purification of expressed recombinant protein. A murine hybridoma designated 4E11 produces a monoclonal antibody that binds the FLAG® peptide in the presence of certain divalent metal cations, as described in U.S. Patent 5,011,912. The 4E11 hybridoma cell line has been deposited with the American Type Culture Collection under accession no. HB 9259. Monoclonal antibodies that bind the FLAG® peptide are available from Eastman Kodak Co., Scientific Imaging Systems Division, New Haven, Connecticut.

Among the variant polypeptides provided herein are variants of native polypeptides that retain the native biological activity or the substantial equivalent thereof. One example is a variant that binds with essentially the same binding affinity as does the native form. Binding affinity can be measured by conventional procedures, e.g., as described in U.S. Patent No. 5,512,457 and as set forth below.

Variants include polypeptides that are substantially homologous to the native form, but which have an amino acid sequence different from that of the native form because of one or more deletions, insertions or substitutions. Particular embodiments include, but are not limited to, polypeptides that comprise from one to ten deletions, insertions or substitutions of amino acid residues, when compared to a native sequence.

A given amino acid may be replaced, for example, by a residue having similar physiochemical characteristics. Examples of such conservative substitutions include substitution of one aliphatic residue for another, such as Ile, Val, Leu, or Ala for one another; substitutions of one polar residue for another, such as between Lys and Arg, Glu and Asp, or Gln and Asn; or substitutions of one aromatic residue for another, such as Phe, Trp, or Tyr for one another. Other conservative substitutions, *e.g*., involving substitutions of entire regions having similar hydrophobicity characteristics, are well known.

Similarly, the DNAs of the invention include variants that differ from a native DNA sequence because of one or more deletions, insertions or substitutions, but that encode a biologically active polypeptide.

The invention further includes polypeptides of the invention with or without associated native-pattern glycosylation. Polypeptides expressed in yeast or mammalian expression systems (*e.g.*, COS-1 or COS-7 cells) can be similar to or -significantly different from a native polypeptide in molecular weight and glycosylation pattern, depending upon the choice of expression system. Expression of polypeptides of the invention in bacterial expression systems, such as *E. coli,* provides non-glycosylated molecules. Further, a given preparation may include multiple differentially glycosylated species of the protein. Glycosyl groups can be removed through conventional methods, in particular those utilizing glycopeptidase. In general, glycosylated polypeptides of the invention can be incubated with a molar excess of glycopeptidase (Boehringer Mannheim).

Correspondingly, similar DNA constructs that encode various additions or substitutions of amino acid residues or sequences, or deletions of terminal or internal residues or sequences are encompassed by the invention. For example, N-glycosylation sites in the polypeptide extracellular domain can be modified to preclude glycosylation, allowing expression of a reduced carbohydrate analog in mammalian and yeast expression systems. N-glycosylation sites in eukaryotic polypeptides are characterized by an amino acid triplet Asn-X-Y, wherein X is any amino acid except Pro and Y is Ser or Thr. Appropriate substitutions, additions, or deletions to the nucleotide sequence encoding these triplets will result in prevention of attachment of carbohydrate residues at the Asn side chain. Alteration of a single nucleotide, chosen so that Asn is replaced by a different amino acid, for example, is sufficient to inactivate an N-glycosylation site. Alternatively, the Ser or Thr can by replaced with another amino acid, such as Ala. Known procedures for inactivating N-glycosylation sites in proteins include those described in U.S. Patent 5,071,972 and EP 276,846,

In another example of variants, sequences encoding Cys residues that are not essential for biological activity can be altered to cause the Cys residues to be deleted or replaced with other amino acids, preventing formation of incorrect intramolecular disulfide bridges upon folding or renaturation.

Other variants are prepared by modification of adjacent dibasic amino acid residues, to enhance expression in yeast systems in which KEX2 protease activity is present. EP 212,914 discloses the use of site-specific mutagenesis to inactivate KEX2 protease processing sites in a protein. KEX2 protease processing sites are inactivated by deleting, adding-orsubstituting-r-esidues to alter Arg-Arg, Arg-Lys, and Lys-Arg pairs to eliminate the occurrence of these adjacent basic residues. Lys-Lys pairings are considerably less susceptible to KEX2 cleavage, and conversion of Arg-Lys or Lys-Arg to Lys-Lys represents a conservative and preferred approach to inactivating KEX2 sites.

### Oligomers

Encompassed by the invention are oligomers or fusion proteins that contain IL-1 zeta, TDZ.1 or TDZ.2 polypeptides. When the polypeptide of the invention is a type I membrane protein, the fusion partner is linked to the C-terminus of the type I membrane protein. Such oligomers may be in the form of covalently-linked or non-covalently-linked multimers, including dimers, trimers, or higher oligomers. As noted above, preferred polypeptides are soluble and thus these oligomers may comprise soluble polypeptides. In one aspect of the invention, the oligomers maintain the binding ability of the polypeptide components and provide therefor, bivalent, trivalent, etc., binding sites.

One embodiment of the invention is directed to oligomers comprising multiple polypeptides joined *via* covalent or non-covalent interactions between peptide moieties fused to the polypeptides. Such peptides may be peptide linkers (spacers), or peptides that have the property of promoting oligomerization. Leucine zippers and certain polypeptides derived from antibodies are among the peptides that can promote oligomerization of the polypeptides attached thereto, as described in more detail below.

### Immunoglobulin-based Oligomers

As one alternative, an oligomer is prepared using polypeptides derived from immunoglobulins. Preparation of fusion proteins comprising certain heterologous polypeptides fused to various portions of antibody-derived polypeptides (including the Fc domain) has been described, e.g., by Ashkenazi et al., *PNAS USA* 88:10535, 1991; Byrn et al., *Nature* 344:677, 1990; and Hollenbaugh and Aruffo, "Construction of Immunoglobulin Fusion Proteins," in Current Protocols in Immunology, Suppl. 4, pp. 10.19.1-10.19.11,1992.

One embodiment of the present invention is directed to a dimer comprising two fusion proteins created by fusing a polypeptide of the invention to an Fc polypeptide derived from an antibody. A gene fusion encoding the polypeptide/Fc fusion protein is inserted into an appropriate expression vector. Polypeptide/Fc fusion proteins are expressed in host cells transformed with the recombinant expression vector, and allowed to assemble much like antibody molecules, whereupon interchain disulfide bonds form between the Fc moieties to yield divalent molecules.

The term "Fc polypeptide" as used herein includes native and mutein forms of polypeptides made up of the Fc region of an antibody comprising any or all of the CH domains of the Fc region. Truncated forms of such polypeptides containing the hinge region that promotes dimerization are also included. Preferred polypeptides comprise an Fc polypeptide derived from a human IgGI antibody.

One suitable Fc polypeptide, described in PCT application WO 93/10151, is a single chain polypeptide extending from the N-terminal hinge region to the native C-terminus of the Fc region of a human IgGI antibody. Another useful Fc polypeptide is the Fc mutein described in U.S. Patent 5,457,035 and in Baum et al., *EMBD J.* 13:3992-4001, 1994.

The amino acid sequence of this mutein is identical to that of the native Fc sequence presented in WO 93/10151, except that amino acid 19 has been changed from Leu to Ala, amino acid 20 has been changed from Leu to Glu, and amino acid 22 has been changed from Gly to Ala. The mutein exhibits reduced affinity for Fc receptors.

The above-described fusion proteins comprising Fc moieties (and oligomers formed therefrom) offer the advantage of facile purification by affinity chromatography over Protein A or Protein G columns.

In other embodiments, the polypeptides of the invention may be substituted for the variable portion of an antibody heavy or light chain. If fusion proteins are made with both heavy and light chains of an antibody, it is possible to form an oligomer with as many as four polypeptide extracellular regions.

### Peptide-linker-Based Oligomers

Alternatively, the oligomer is a fusion protein comprising multiple polypeptides, with or without peptide linkers (spacer peptides). Among the suitable peptide linkers are those described in U.S. Patents 4,751,180 and 4,935,233,

A DNA sequence encoding a desired peptide linker may be inserted between, and in the same reading frame as, the DNA sequences of the invention, using any suitable conventional technique. For example, a chemically synthesized oligonucleotide encoding the linker may be ligated between the sequences. In particular embodiments, a fusion protein comprises from two to four soluble polypeptides of the invention, separated by peptide linkers.

### Leucine-Zippers

Another method for preparing the oligomers of the invention involves use of a leucine zipper. Leucine zipper domains are peptides that promote oligomerization of the proteins in which they are found. Leucine zippers were originally identified in several DNA-binding proteins (Landschulz et al., *Science* 240:1759, 1988), and have since been found in a variety of different proteins. Among the known leucine zippers are naturally occurring peptides and derivatives thereof that dimerize or trimerize.

The zipper domain (also referred to herein as an oligomerizing, or oligomer-forming, domain) comprises a repetitive heptad repeat, often with four or five leucine residues interspersed with other amino acids. Examples of zipper domains are those found in the yeast transcription factor GCN4 and a heat-stable DNA-binding protein found in rat liver (C/EBP; Landschulz et al., *Science,* 243:1681, 1989). Two nuclear transforming proteins, *fos* and *jun,* also exhibit zipper domains, as does the gene product of the murine proto-oncogene, c-myc (Landschulz et al., *Science,* 240:1759, 1988). The products of the nuclear oncogenes *fos* and *jun* comprise zipper domains that preferentially form heterodimers (O'Shea et al., *Science,* 245:646, 1989; Turner et al., *Science,* 243:1689, 1989). The zipper domain is necessary for biological activity (DNA binding) in these proteins.

The fusogenic proteins of several different viruses, including paramyxovirus, coronavirus, measles virus and many retroviruses, also possess zipper domains (Buckland et al., *Nature,* 338:547,1989; Britton, *Nature,* 353:394, 1991; Delwart and Mosialos, *AIDS Research and Human Retroviruses,* 6:703, 1990). The zipper domains in these fusogenic viral proteins are near the transmembrane region of the proteins; it has been suggested that the zipper domains could contribute to the oligomeric structure of the fusogenic proteins. Oligomerization of fusogenic viral proteins is involved in fusion pore formation (Spruce et al, *Proc. Natl. Acad Sci. U.S.A.,* 88:3523, 1991). Zipper domains have also been recently reported to play a role in oligomerization of heat-shock transcription factors (Rabindran et al., *Science,* 259:230, 1993).

Zipper domains fold as short, parallel coiled coils. (O'Shea et al., *Science,* 254:539, 1991) The general architecture of the parallel coiled coil has been well characterized, with a "knobs-into-holes" packing as proposed by Crick in 1953 (Crick, *Acta Crystallogr.* 6:689, 1953). The dimer formed by a zipper domain is stabilized by the heptad repeat, designated *(abcdefg)*_{*n*} according to the notation of McLachlan and Stewart, *J. Mol. Biol.,* 98:293, 1975, in which residues a and d are generally hydrophobic residues, with *d* being a leucine, which line up on the same face of a helix. Oppositely-charged residues commonly occur at positions *g* and *e*. Thus, in a parallel coiled coil formed from two helical zipper domains, the "knobs" formed by the hydrophobic side chains of the first helix are packed into the "holes" formed between the side chains of the second helix.

The residues at position *d* (often leucine) contribute large hydrophobic stabilization energies, and are important for oligomer formation (Krystek et al., *Int. J. Peptide Res.,* 38:229, 1991). Lovejoy et al., *Science,* 259:1288, 1993, recently reported the synthesis of a triple-stranded α-helical bundle in which the helices run up-up-down. Their studies confirmed that hydrophobic stabilization energy provides the main driving force for the formation of coiled coils from helical monomers. These studies also indicate that electrostatic interactions contribute to the stoichiometry and geometry of coiled coils. Further discussion of the structure of leucine zippers is found in Harbury et al., *Science,* 262:1401, 1993.

Examples of leucine zipper domains suitable for producing soluble oligomeric proteins are described in PCT application WO-94/10308, and the leucine zipper derived from lung surfactant protein D (SPD) described in Hoppe et al., *FEBS Letters,* 344:191, 1994. The use of a modified leucine zipper that allows for stable trimerization of a heterologous protein fused thereto is described in Fanslow et al., *Semin. Immunol.,* 6:267-278, 1994. Recombinant fusion proteins comprising a soluble polypeptide fused to a leucine zipper peptide are expressed in suitable host cells, and the soluble oligomer that forms is recovered from the culture supernatant.

Certain leucine zipper moieties preferentially form trimers. One example is a leucine zipper derived from lung surfactant protein D (SPD), as described in Hoppe et al., *FEBS Letters,* 344:191, 1994, and in U.S. Patent 5,716,805.

This lung SPD-derived leucine zipper peptide comprises the amino acid sequence Pro Asp Val Ala Ser Leu Arg Gln Gln Val Glu Ala Leu Gln Gly Gin Val Gln His Leu Gin Ala Ala Phe Ser Gin Tyr (SEQ ID NO:12).

Another example of a leucine zipper that promotes trimerization is a peptide comprising the amino acid sequence Arg Met Lys Gln Ile Glu Asp Lys Ile Glu Glu Ile Leu Ser Lys Ile Tyr His Ile Glu Asn Glu Ile Ala Arg Ile Lys Lys Leu Ile Gly Glu Arg (SEQ ID NO: 13), as described in U.S. Patent 5,716,805. In one alternative embodiment, an N-terminal Asp residue is added; in another, the peptide lacks the N-terminal Arg residue.

Fragments of the foregoing zipper peptides that retain the property of promoting oligomerization may be employed as well. Examples of such fragments include, but are not limited to, peptides lacking one or two of the N-terminal or C-terminal residues presented in the foregoing amino acid sequences. Leucine zippers may be derived from naturally occurring leucine zipper peptides, e.g., *via* conservative substitution(s) in the native amino acid sequence, wherein the peptide's ability to promote oligomerization is retained.

Other peptides derived from naturally occurring trimeric proteins may be employed in preparing trimeric oligomers. Alternatively, synthetic peptides that promote oligomerization may be employed. In particular embodiments, leucine residues in a leucine zipper moiety are replaced by isoleucine residues. Such peptides comprising isoleucine may be referred to as isoleucine zippers, but are encompassed by the term "leucine zippers" as employed herein.

### PRODUCTION OF POLYPEPTIDES AND FRAGMENTS THEREOF

Expression, isolation and purification of the polypeptides and fragments of the invention may be accomplished by any suitable technique, including but not limited to the following:

### Expression Systems

The present invention also provides recombinant cloning and expression vectors containing DNA, as well as host cell containing the recombinant vectors. Expression vectors comprising DNA may be used to prepare the polypeptides or fragments of the invention encoded by the DNA. A method for producing polypeptides comprises culturing host cells transformed with a recombinant expression vector encoding the polypeptide, under conditions that promote expression of the polypeptide, then recovering the expressed polypeptides from the culture. The skilled artisan will recognize that the procedure for purifying the expressed polypeptides will vary according to such factors as the type of host cells employed, and whether the polypeptide is membrane-bound or a soluble form that is secreted from the host cell.

Any suitable expression system may be employed. The vectors include a DNA encoding a polypeptide or fragment of the invention, operably linked to suitable transcriptional or translational regulatory nucleotide sequences, such as those derived from a mammalian, microbial, viral, or insect gene. Examples of regulatory sequences include transcriptional promoters, operators, or enhancers, an mRNA ribosomal binding site, and appropriate sequences which control transcription and translation initiation and termination. Nucleotide sequences are operably linked when the regulatory sequence functionally relates to the DNA sequence. Thus, a promoter nucleotide sequence is operably linked to a DNA sequence if the promoter nucleotide sequence controls the transcription of the DNA sequence. An origin of replication that confers the ability to replicate in the desired host cells, and a selection gene by which transformants are identified, are generally incorporated into the expression vector.

In addition, a sequence encoding an appropriate signal peptide (native or heterologous) can be incorporated into expression vectors. A DNA sequence for a signal peptide (secretory leader) may be fused in frame to the nucleic acid sequence of the invention so that the DNA is initially transcribed, and the mRNA translated, into a fusion protein comprising the signal peptide. A signal peptide that is functional in the intended host cells promotes extracellular secretion of the polypeptide. The signal peptide is cleaved from the polypeptide upon secretion of polypeptide from the cell.

The skilled artisan will also recognize that the position(s) at which the signal peptide is cleaved may differ from that predicted by computer program, and may vary according to such factors as the type of host cells employed in expressing a recombinant polypeptide. A protein preparation may include a mixture of protein molecules having different N-terminal amino acids, resulting from cleavage of the signal peptide at more than one site. Particular examples of mature proteins include, but are not limited to, proteins having the residue at position 6,23,25,26,39,41, or 48 of SEQ ID NO:3 and at position 1 or 19 of SEQ ID NO:4 as the N-terminal amino acid.

Suitable host cells for expression of polypeptides include prokaryotes, yeast or higher eukaryotic cells. Mammalian or insect cells are generally preferred for use as host cells. Appropriate cloning and expression vectors for use with bacterial, fungal, yeast, and mammalian cellular hosts are described, for example, in Pouwels et al., Cloning Vectors: A Laboratory Manual, Elsevier, New York,1985. Cell-free translation systems could also be employed to produce polypeptides using RNAs derived from DNA constructs disclosed herein.

### Prokaryotic Systems

Prokaryotes include gram-negative or gram-positive organisms. Suitable prokaryotic host cells for transformation include, for example, *E. coli, Bacillus subtilis, Salmonella typhimurium,* and various other species within the genera *Pseudomonas, Streptomyces,* and *Staphylococcus.* In a prokaryotic host cell, such as *E. coli,* a polypeptide may include an N-terminal methionine residue to facilitate expression of the recombinant polypeptide in the prokaryotic host cell. The N-terminal Met may be cleaved from the expressed recombinant polypeptide.

Expression vectors for use in prokaryotic host cells generally comprise one or more phenotypic selectable marker genes. A phenotypic selectable marker gene is, for example, a gene encoding a protein that confers antibiotic resistance or that supplies an autotrophic requirement. Examples of useful expression vectors for prokaryotic host cells include those derived from commercially available plasmids such as the cloning vector pBR322 (ATCC 37017). pBR322 contains genes for ampicillin and tetracycline resistance and thus provides simple means for identifying transformed cells. An appropriate promoter and a DNA sequence are inserted into the pBR322 vector. Other commercially available vectors include, for example, pKK223-3 (Pharmacia Fine Chemicals, Uppsala, Sweden) and pGEM1 (Promega Biotec, Madison, WI, USA).

Promoter sequences commonly used for recombinant prokaryotic host cell expression vectors include β-lactamase (penicillinase), lactose promoter system (Chang et al., *Nature,* 275:615, 1978; and Goeddel et al., *Nature,* 281:544, 1979), tryptophan (trp) promoter system (Goeddel et al., *Nucl. Acids Res.,* 8:4057, 1980; and EP-A-36776) and tac promoter (Maniatis, Molecular Cloning: A Laboratory Manual, p. 412, Cold Spring Harbor Laboratory, 1982). A particularly useful prokaryotic host cell expression system employs a phage λP_{L} promoter and a cI857ts thermolabile repressor sequence. Plasmid vectors available from the American Type Culture Collection which incorporate derivatives of the λP_{L} promoter include plasmid pHUB2 (resident in *E*. *coli* strain JMB9, ATCC 37092) and pPLc28 (resident in *E. coli* RR1, ATCC 53082).

### Yeast Systems

Alternatively, the polypeptides may be expressed in yeast host cells, preferably from the *Saccharomyces* genus *(e.g., S. cerevisiae,).* Other genera of yeast, such as *Pichia or Kluyveromyces,* may also-be-employed. Yeast vectors will often contain an origin of replication sequence from a 2µ yeast plasmid, an autonomously replicating sequence (ARS), a promoter region, sequences for polyadenylation, sequences for transcription termination, and a selectable marker gene. Suitable promoter sequences for yeast vectors include, among others, promoters for metallothionein, 3-phosphoglycerate kinase (Hitzeman et al., *J. Biol. Chem.,* 255:2073, 1980) or other glycolytic enzymes (Hess et al., *J Adv. Enzyme Reg.,* 7:149, 1968; and Holland et al., *Biochem.* 17:4900, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phospho-glucose isomerase, and glucokinase. Other suitable vectors and promoters for use in yeast expression are further described in Hitzeman, EPA-73,657. Another alternative is the glucose-repressible ADH2 promoter described by Russell et al., *J. Biol. Chem.,* 258:2674, 1982; and Beier et al., *Nature,* 300:724, 1982. Shuttle vectors replicable in both yeast and *E*. *coli* may be constructed by inserting DNA sequences from pBR322 for selection and replication in *E*. *coli* (Amp^{r} gene and origin of replication) into the above-described yeast vectors.

The yeast α-factor leader sequence may be employed to direct secretion of the polypeptide. The α-factor leader sequence is often inserted between the promoter sequence and the structural gene sequence. (Kurjan et al., *Cell,* 30:933, 1982; and Bitter et al., *Proc. Natl. Acad Sci. USA,* 81:5330, 1984.) Other leader sequences suitable for facilitating secretion of recombinant polypeptides from yeast hosts are known to those of skill in the art. A leader sequence may be modified near its 3' end to contain one or more restriction sites. This will facilitate fusion of the leader sequence to the structural gene.

Yeast transformation protocols are known to those of skill in the art. One such protocol is described by Hinnen et al., *Proc. Natl. Acad Sci. USA,* 75:1929, 1978. The Hinnen et al. protocol selects for Trp⁺ transformants in a selective medium, wherein the selective medium consists of 0.67% yeast nitrogen base, 0.5% casamino acids, 2% glucose, 10 mg/ml adenine and 20 mg/ml uracil.

Yeast host cells transformed by vectors containing an ADH2 promoter sequence may be grown for inducing expression in a "rich" medium. An example of a rich medium is one consisting of 1% yeast extract, 2% peptone, and 1% glucose supplemented with 80 mg/ml adenine and 80 mg/ml uracil. Derepression of the ADH2 promoter occurs when glucose is exhausted from the medium.

### Mammalian or Insect Systems

Mammalian or insect host cell culture systems also may be employed to express recombinant polypeptides. Bacculovirus systems for production of heterologous proteins in insect cells are reviewed by Luckow et al., *Bio*/*Technology,* 6:47, 1988. Established cell lines of mammalian origin also may be employed. Examples of suitable mammalian host cell lines include the COS-7 line of monkey kidney cells (ATCC CRL 1651) (Gluzman et al., *Cell,* 23:175, 1981), L cells, C127 cells, 3T3 cells (ATCC CCL 163), Chinese hamster ovary (CHO) cells, HeLa cells, and BHK (ATCC CRL 10) cell lines, and the CV1/EBNA cell line derived from the African green monkey kidney cell line CV 1 (ATCC CCL 70) as described by McMahan et al., *EMBO J.,* 10: 2821, 1991.

Established methods for introducing DNA into mammalian cells have been described by Kaufman, R.J., Large Scale Mammalian Cell Culture, pp. 15-69,1990. Additional protocols using commercially available reagents, such as Lipofectamine lipid reagent (Gibco/BRL) or Lipofectamine-Plus lipid reagent, can be used to transfect cells (Felgner et al., *Proc. Natl. Acad Sci. USA,* 84:7413-7417, 1987). In addition, electroporation can be used to transfect mammalian cells using conventional procedures, such as those in Sambrook et al., Molecular Cloning: A Laboratory Manual, 2nd ed. Vol. 1-3, Cold Spring Harbor Laboratory Press, 1989. Selection of stable transformants can be performed using methods known in the art, such as, for example, resistance to cytotoxic drugs. Kaufman et al., *Meth. in Enzymology,* 185:487-511, 1990, describes several selection schemes, such as dihydrofolate reductase (DHFR) resistance. A suitable host strain for DHFR selection can be CHO strain DX-B11, which is deficient in DHFR (Urlaub et al., *Proc. Natl. Acad Sci. USA,* 77:4216-4220, 1980). A plasmid expressing the DHFR cDNA can be introduced into strain DX-B11, and only cells that contain the plasmid can grow in the appropriate selective media. Other examples of selectable markers that can be incorporated into an expression vector include cDNAs conferring resistance to antibiotics, such as G418 and hygromycin B. Cells harboring the vector can be selected on the basis of resistance to these compounds.

Transcriptional and translational control sequences for mammalian host cell expression vectors can be excised from viral genomes. Commonly used promoter sequences and enhancer sequences are derived from polyoma virus, adenovirus 2, simian virus 40 (SV40), and human cytomegalovirus. DNA sequences derived from the SV40 viral genome, for example, SV40 origin, early and late promoter, enhancer, splice, and polyadenylation sites can be used to provide other genetic elements for expression of a structural gene sequence in a mammalian host cell. Viral early and late promoters are particularly useful because both are easily obtained from a viral genome as a fragment, which can also contain a viral origin of replication (Fiers et al., *Nature,* 273:113, 1978; and Kaufinan, *Meth. in Enzymology,* 1990). Smaller or larger SV40 fragments can also be used, provided the approximately 250 bp sequence extending from the *Hind* III site toward the *Bgl* I site located in the SV40 viral origin of replication site is included.

Additional control sequences shown to improve expression of heterologous genes from mammalian expression vectors include such elements as the expression augmenting sequence element (EASE) derived from CHO cells (Morris et al., Animal Cell Technology. pp. 529-534, 1997; and PCT Application WO 97/25420) and the tripartite leader (TPL) and VA gene RNAs from Adenovirus 2 (Gingeras et al., J. *Biol. Chem.,* 257:13475-13491, 1982). The internal ribosome entry site (IRES) sequences of viral origin allows dicistronic mRNAs to be translated efficiently (Oh et al., *Current Opinion in Genetics and Development,* 3:295-300, 1993; and Ramesh et al., *Nucleic Acids Research,* 24:2697-2700, 1996). Expression of a heterologous cDNA as part of a dicistronic mRNA followed by the gene for a selectable marker (e.g. DHFR) has been shown to improve transfectability of the host and expression of the heterologous cDNA (Kaufman, *Meth. in Enzymology,* 1990). Exemplary expression vectors that employ dicistronic mRNAs are pTR-DGGFP described by Mosser et al., *Biotechniques,* 22:150-161, 1997, and p2A51 described by Morris et al., *Animal Cell Technology,* pp. 529-534, 1997.

A useful high expression vector, pCAVNOT, has been described by Mosley et al., *Cell,* 59:335-348, 1989. Other expression vectors for use in mammalian host cells can be constructed as disclosed by Okayama et al., *(Mol. Cell. Biol.,* 3:280, 1983. A useful system for stable high level expression of mammalian cDNAs in C 127 murine mammary epithelial cells can be constructed substantially as described by Cosman et al., *Mol. Immunol.,* 23:935,1986. A useful high expression vector, PMLSV N1/N4, described by Cosman et al., *Nature,* 312:768, 1984, has been deposited as ATCC 39890. Additional useful mammalian expression vectors are described in EP-A-0367566, and in WO 91/18982. In yet another alternative, the vectors can be derived from retroviruses.

Another useful expression vector, pFLAG® , can be used. FLAG® technology is centered on the fusion of a low molecular weight (1kD), hydrophilic, FLAG® marker peptide to the N-terminus of a recombinant protein expressed by pFLAG® expression vectors. pDC31 is another specialized vector used for expressing proteins in CHO cells. pDC311 is characterized by a bicistronic sequence containing the gene of interest and a dihydrofolate reductase (DHFR) gene with an internal ribosome binding site for DHFR translation, an expression augmenting sequence element (EASE), the human CMV promoter, a tripartite leader sequence, and a polyadenylation site.

Regarding signal peptides that may be employed, the native signal peptide may be replaced by a heterologous signal peptide or leader sequence, if desired. The choice of signal peptide or leader may depend on factors such as the type of host cells in which the recombinant polypeptide is to be produced. To illustrate, examples of heterologous signal peptides that are functional in mammalian host cells include the signal sequence for interleukin-7 (IL-7) described in United States Patent 4,965, 195; the signal sequence for interleukin-2 receptor described in Cosman et al., *Nature,* 312:768, 1984; the interleukin-4 receptor signal peptide described in EP 367,566; the type I interleukin-1 receptor signal peptide described in U.S. Patent 4,968,607; and the type II interleukin-1 receptor signal peptide described in EP 460,846.

### Purification

The invention also includes methods of isolating and purifying the polypeptides and fragments thereof.

### Isolation and Purification

The "isolated" polypeptides or fragments thereof encompassed by this invention are polypeptides or fragments that are not in an environment identical to an environment in which it or they can be found in nature. The "purified" polypeptides or fragments thereof encompassed by this invention are essentially free of association with other proteins or polypeptides, for example, as a purification product of recombinant expression systems such as those described above or as a purified product from a non-recombinant source such as naturally occurring cells and/or tissues.

In one preferred embodiment, the purification of recombinant polypeptides or fragments can be accomplished using fusions of polypeptides or fragments of the invention to another polypeptide to aid in the purification of polypeptides or fragments of the invention. Such fusion partners can include the poly-His or other antigenic identification peptides described above as well as the Fc moieties described previously.

With respect to any type of host cell, as is known to the skilled artisan, procedures for purifying a recombinant polypeptide or fragment will vary according to such factors as the type of host cells employed and whether or not the recombinant polypeptide or fragment is secreted into the culture medium.

In general, the recombinant polypeptide or fragment can be isolated from the host cells if not secreted, or from the medium or supernatant if soluble and secreted, followed by one or more concentration, salting-out, ion exchange, hydrophobic interaction, affinity purification or size exclusion chromatography steps. As to specific ways to accomplish these steps, the culture medium first can be concentrated using a commercially available protein concentration filter, for example, an Amicon or Millipore Pellicon ultrafiltration unit. Following the concentration step, the concentrate can be applied to a purification matrix such as a gel filtration medium. Alternatively, an anion exchange resin can be employed, for example, a matrix or substrate having pendant diethylaminoethyl (DEAE) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups. In addition, a chromatofocusing step can be employed. Alternatively, a hydrophobic interaction chromatography step can be employed. Suitable matrices can be phenyl or octyl moieties bound to resins. In addition, affinity chromatography with a matrix which selectively binds the recombinant protein can be employed. Examples of such resins employed are lectin columns, dye columns, and metal-chelating columns. Finally, one or more reverse-phase high performance liquid chromatography (RP-HPLC) steps employing hydrophobic RP-HPLC media, (e.g., silica gel or polymer resin having pendant methyl, octyl, octyldecyl or other aliphatic groups) can be employed to further purify the polypeptides. Some or all of the foregoing purification steps, in various combinations, are well known and can be employed to provide an isolated and purified recombinant protein.

It is also possible to utilize an affinity column comprising a polypeptide-binding protein of the invention, such as a monoclonal antibody generated against polypeptides of the invention, to affinity-purify expressed polypeptides. These polypeptides can be removed from an affinity column using conventional techniques, e.g., in a high salt elution buffer and then dialyzed into a lower salt buffer for use or by changing pH or other components depending on the affinity matrix utilized, or be competitively removed using the naturally occurring substrate of the affinity moiety, such as a polypeptide derived from the invention.

In this aspect of the invention, polypeptide-binding proteins, such as the anti-polypeptide antibodies of the invention or other proteins that may interact with the polypeptide of the invention, can be bound to a solid phase support such as a column chromatography matrix or a similar substrate suitable for identifying, separating, or purifying cells that express polypeptides of the invention on their surface. Adherence of polypeptide-binding proteins of the invention to a solid phase contacting surface can be accomplished by any means. For example, magnetic microspheres can be coated with these polypeptide-binding proteins and held in the incubation vessel through a magnetic field. Suspensions of cell mixtures are contacted with the solid phase that has such polypeptide-binding proteins thereon. Cells having polypeptides of the invention on their surface bind to the fixed polypeptide-binding protein and unbound cells then are washed away. This affinity-binding method is useful for purifying, screening, or separating such polypeptide-expressing cells from solution. Methods of releasing positively selected cells from the solid phase are known in the art and encompass, for example, the use of enzymes. Such enzymes are preferably non-toxic and non-injurious to the cells and are preferably directed to cleaving the cell-surface binding partner.

Alternatively, mixtures of cells suspected of containing polypeptide-expressing cells of the invention first can be incubated with a biotinylated polypeptide-binding protein of the invention. Incubation periods are typically at least one hour in duration to ensure sufficient binding to polypeptides of the invention. The resulting mixture then is passed through a column packed with avidin-coated beads, whereby the high affinity of biotin for avidin provides the binding of the polypeptide-binding cells to the beads. Use of avidin-coated beads is known in the art (Berenson et al., *J*. *Cell. Biochem.,* 10D:239, 1986). Wash of unbound material and the release of the bound cells is performed using conventional methods.

The desired degree of purity depends on the intended use of the protein. A relatively high degree of purity is desired when the polypeptide is to be administered *in vivo,* for example. In such a case, the polypeptides are purified such that no protein bands corresponding to other proteins are detectable upon analysis by SDS-polyacrylamide gel electrophoresis (SDS-PAGE). It will be recognized by one skilled in the pertinent field that multiple bands corresponding to the polypeptide may be visualized by SDS-PAGE, due to differential glycosylation, differential post-translational processing, and the like. Most preferably, the polypeptide of the invention is purified to substantial homogeneity, as indicated by a single protein band upon analysis by SDS-PAGE. The protein band may be visualized by silver staining, Coomassie blue staining, or (if the protein is radiolabeled) by autoradiography.

### Assays

The purified polypeptides of the invention (including proteins, polypeptides, fragments, variants, oligomers, and other forms) may be tested for the ability to bind the binding partner in any suitable assay, such as a conventional binding assay. To illustrate, the polypeptide may be labeled with a detectable reagent (e.g., a radionuclide, chromophore, enzyme that catalyzes a colorimetric or fluorometric reaction, and the like). The labeled polypeptide is contacted with cells expressing the binding partner. The cells then are washed to remove unbound labeled polypeptide, and the presence of cell-bound label is determined by a suitable technique, chosen according to the nature of the label.

One example of a binding assay procedure is as follows. A recombinant expression vector containing the binding partner cDNA is constructed using methods well known in the art. CV1-EBNA-1 cells in 10 cm² dishes are transfected with the recombinant expression vector. CV-1/EBNA-1 cells (ATCC CRL 10478) constitutively express EBV nuclear antigen-1 driven from the CMV immediate-early enhancer/promoter. CV1-EBNA-1 was derived from the African Green Monkey kidney cell line CV-1 (ATCC CCL 70), as described by McMahan et al. *(EMBO J.* 10:2821, 1991).

The transfected cells are cultured for 24 hours, and the cells in each dish then are split into a 24-well plate. After culturing an additional 48 hours, the transfected cells (about 4 x 10⁴ cells/well) are washed with BM-NFDM, which is binding medium (RPMI 1640 containing 25 mg/ml bovine serum albumin, 2 mg/ml sodium azide, 20 mM Hepes pH 7.2) to which 50 mg/ml nonfat dry milk has been added. The cells then are incubated for 1 hour at 37 C with various concentrations of, for example, a soluble polypeptide/Fc fusion protein made as set forth above. Cells then are washed and incubated with a constant saturating concentration of a¹²⁵I-mouse anti-human IgG in binding medium, with gentle agitation for 1 hour at 37 C. After extensive washing, cells are released *via* trypsinization.

The mouse anti-human IgG employed above is directed against the Fc region of human IgG and can be obtained from Jackson Immunoresearch Laboratories, Inc., West Grove, PA. The antibody is radioiodinated using the standard chloramine-T method. The antibody will bind to the Fc portion of any polypeptide/Fc protein that has bound to the cells. In all assays, non-specific binding of ¹²⁵I-antibody is assayed in the absence of the Fc fusion protein/Fc, as well as in the presence of the Fc fusion protein and a 200-fold molar excess of unlabeled mouse anti-human IgG antibody.

Cell-bound ¹²⁵I-antibody is quantified on a Packard Autogamma counter. Affinity calculations (Scatchard, *Ann. N.Y Acad Sci.,* 51:660, 1949) are generated on RS/1 (BBN Software, Boston, MA) run on a Microvax computer.

Another type of suitable binding assay is a competitive binding assay. To illustrate, biological activity of a variant may be determined by assaying for the variant's ability to compete with the native protein for binding to the binding partner.

Competitive binding assays can be performed by conventional methodology. Reagents that may be employed in competitive binding assays include radiolabeled polypeptides of the invention and intact cells expressing the binding partner (endogenous or recombinant). For example, a radiolabeled soluble IL-1 zeta fragment can be used to compete with a soluble IL-1 zeta variant for binding to cell surface IL-1 zeta receptors. Instead of intact cells, one could substitute a soluble binding partner/Fc fusion protein bound to a solid phase through the interaction of Protein A or Protein G (on the solid phase) with the Fc moiety. Chromatography columns that contain Protein A and Protein G include those available from Pharmacia Biotech, Inc., Piscataway, NJ.

Another type of competitive binding assay utilizes radiolabeled soluble binding partner, such as a soluble IL-1 zeta receptor/Fc fusion or Xrec2 ligand/Fc fusion protein, and intact cells expressing the binding partner. Qualitative results can be obtained by competitive autoradiographic plate binding assays, while Scatchard plots (Scatchard, *Ann. N. Y. Acad Sci.,* 51:660, 1949) may be utilized to generate quantitative results.

### USE OF IL-1 ZETA, TDZ.1, TDZ.2 TDZ.3 AND XREC2 NUCLEIC ACIDS OR OLIGONUCLEOTIDES

In addition to being used to express polypeptides as described above, the IL-1 zeta, TDZ.1, TDZ.2, TDZ.3 and Xrec2 nucleic acids, including DNA, RNA, mRNA, and oligonucleotides thereof can be used:
- as probes to identify nucleic acid encoding proteins of the IL-1 ligand and receptor families;
- to identify human chromosomes 2 and X;
- to map genes on human chromosomes 2 and X;
- to identify genes associated with certain diseases, syndromes, or other conditions associated with human chromosomes 2 and X;
- as single-stranded sense or antisense oligonucleotides, to inhibit expression of polypeptides encoded by the IL-1 zeta, TDZ.1, TDZ.2, TDZ.3 and Xrec2 genes;
- to help detect defective genes in an individual; and
- for gene therapy.

### Probes

Among the uses of nucleic acids disclosed herein is the use of fragments as probes or primers. Such fragments generally comprise at least about 17, at least 30 or at least 60, contiguous nucleotides of a DNA sequence.

Because homologs of SEQ ID NOs:1,2,5,6 and 7, from other mammalian species, are contemplated herein, probes based on the human DNA sequences of SEQ ID NOs: 1, 2, 5, 6 and 7 may be used to screen cDNA libraries derived from other mammalian species, using conventional cross-species hybridization techniques.

Using knowledge of the genetic code in combination with the amino acid sequences set forth above, sets of degenerate oligonucleotides can be prepared. Such oligonucleotides are useful as primers, e.g., in polymerase chain reactions (PCR), whereby DNA fragments are isolated and amplified.

### Chromosome Mapping

All or a portion of the nucleic acids of IL-1 zeta of SEQ ID NO: or IL-1 zeta splice variants of SEQ ID NOs:5, 6, and 7, including oligonucleotides, can be used by those skilled in the art using-well-known techniques to identify human chromosome 2, as well as the specific locus thereof, that contains the DNA of IL-1 ligand family members. In addition, all or a portion of the nucleic acids of Xrec2 of SEQ ID NO:2, including oligonucleotides, can be used to identify human chromosome X, as well as the specific locus thereof that contains the DNA of IL-1 receptor family members. Useful techniques include, but are hot limited to, using the sequence or portions, including oligonucleotides, as a probe in various well-known techniques such as radiation hybrid mapping (high resolution), in situ hybridization to chromosome spreads (moderate resolution), and Southern blot hybridization to hybrid cell lines containing individual human chromosomes (low resolution).

For example, chromosomes can be mapped by radiation hybridization. PCR is performed using the Whitehead Institute/MIT Center for Genome Research Genebridge4 panel of 93 radiation hybrids (httn://www-genome.wi.mit.edu/ftp/distribution/human STS_releases/july97/rhmap/g enebridge4.html). Primers are used which lie within a putative exon of the gene of interest and which amplify a product from human genomic DNA, but do not amplify hamster genomic DNA. The results of the PCRs are converted into a data vector that is submitted to the Whitehead/MIT Radiation Mapping site on the internet (http://www-seq.wi.mit.edu). The data is scored and the chromosomal assignment and placement relative to known Sequence Tag Site (STS) markers on the radiation hybrid map is provided. The following web site provides additional information about radiation hybrid mapping:
http://www-genome.wi.mit.edu/ftp/distribution/human_STS_releases/july97// 07-97.INTRO.html).

### Identifying Associated Diseases

As set forth below, the nucleic acids of IL-1 zeta of SEQ ID NO:1, and IL- 1 zeta splice variants of SEQ ID NOs: 5, 6, and 7, have been mapped by radiation hybridization and high-throughput-shotgun sequencing to the 2q11-12 region of human chromosome 2. Human chromosome 2 is associated with specific diseases which include but are not limited to glaucoma, ectodermal dysplasia, insulin-dependent diabetes mellitus, wrinkly skin syndrome, T-cell.leukemia/lymphoma, and tibial muscular dystrophy. The nucleic acids of Xrec2 of SEQ ID NO:2 have been mapped by radiation hybridization and high-throughput-shotgun sequencing to the Xp22 region of human chromosome X. Human chromosome X is associated with retinoschisis, lissencephaly, subcortical laminalheteropia, mental retardation, cowchock syndrome, bazex syndrome, hypertrichosis, lymphoproliferative syndrome, immunodeficiency, Langer mesomelic dysplasia, and leukemia. Thus, the nucleic acids of SEQ ID NOs:1, 5, 6, 7, and 2 or a fragment thereof can be used by one skilled in the art using well-known techniques to analyze abnormalities associated with gene mapping to chromosomes 2 and X. This enables one to distinguish conditions in which this marker is rearranged or deleted. In addition, nucleic and molecules of SEQ ID NOs: 1, 2, 5, 6, and 7 or a fragment thereof can be used as a positional marker to map other genes of unknown location.

The DNA may be used in developing treatments for any disorder mediated (directly or indirectly) by defective, or insufficient amounts of, the genes corresponding to the nucleic acids of the invention. Disclosure herein of native nucleotide sequences permits the detection of defective genes, and the replacement thereof with normal genes. Defective genes may be detected in *in vitro* diagnostic assays, and by comparison of a native nucleotide sequence disclosed herein with that of a gene derived from a person suspected of harboring a defect in this gene,

### Sense-Antisense

Other useful fragments of the nucleic acids include antisense or sense oligonucleotides comprising a single-stranded nucleic acid sequence (either RNA or DNA) capable of binding to target mRNA (sense) or DNA (antisense) sequences. Antisense or sense oligonucleotides may comprise a fragment of DNA (SEQ ID NOs: 1, 2, 5, 6 and 7). Such a fragment generally comprises at least about 14 nucleotides, preferably from about 14 to about 30 nucleotides. The ability to derive an antisense or a sense oligonucleotide, based upon a cDNA sequence encoding a given protein is described in, for example, Stein et al., *Cancer Res.,* 48:2659, 1988; and van der Krol et al., *BioTechniques,* 6:958, 1988.

Binding of antisense or sense oligonucleotides to target nucleic acid sequences results in the formation of duplexes that block or inhibit protein expression by one of several means, including enhanced degradation of the mRNA by RNAseH, inhibition of splicing, premature termination of transcription or translation, or by other means. The antisense oligonucleotides thus may be used to block expression of proteins. Antisense or sense oligonucleotides further comprise oligonucleotides having modified sugar-phosphodiester backbones (or other sugar linkages; such as those described in WO91/06629) and wherein such sugar linkages are resistant to endogenous nucleases. Such oligonucleotides with resistant sugar linkages are stable *in vivo* (*i.e.,* capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10448, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, lipofection, CaPO₄-mediated DNA transfection, electroporation, or by using gene transfer vectors such as Epstein-Barr virus.

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

### USE OF IL-1 ZETA. TDZ.1, TDZ.2 TDZ.3 AND XREC2 POLYPEPTIDES AND FRAGMENTED POLYPEPTIDES

Uses include, but are not limited to, the following:
- Purifying proteins and measuring activity thereof
- Delivery Agents
- Therapeutic and Research Reagents
- Controls for peptide fragmentation
- Identification of unknown proteins
- Preparation of Antibodies

### Purification Reagents

Each of the polypeptides of the invention finds use as a protein purification reagent. The polypeptides may be attached to a solid support material and used to purify the binding partner proteins by affinity chromatography. In particular embodiments, a polypeptide (in any form described herein that is capable of binding the binding partner) is attached to a solid support by conventional procedures. As one example, chromatography columns containing functional groups that will react with functional groups on amino acid side chains of proteins are available (Pharmacia Biotech, Inc., Piscataway, NJ). In an alternative, a polypeptide/Fc protein (as discussed above) is attached to Protein A- or Protein G-containing chromatography columns through interaction with the Fc moiety.

The polypeptide also finds use in purifying or identifying cells that express the binding partner on the cell surface. Polypeptides are bound to a solid phase such as a column chromatography matrix or a similar suitable substrate. For example, magnetic microspheres can be coated with the polypeptides and held in an incubation vessel through a magnetic field. Suspensions of cell mixtures containing the binding partner expressing cells are contacted with the solid phase having the polypeptides thereon. Cells expressing the binding partner on the cell surface bind to the fixed polypeptides, and unbound cells then are washed away.

Alternatively, the polypeptides can be conjugated to a detectable moiety, then incubated with cells to be tested for binding partner expression. After incubation, unbound labeled matter is removed and the presence or absence of the detectable moiety on the cells is determined.

In a further alternative, mixtures of cells suspected of containing cells expressing the binding partner are incubated with biotinylated polypeptides. Incubation periods are typically at least one hour in duration to ensure sufficient binding. The resulting mixture then is passed through a column packed with avidin-coated beads, whereby the high affinity of biotin for avidin provides binding of the desired cells to the beads. Procedures for using avidin-coated beads are known (Berenson et al., *J. Cell. Biochem.,* 10D:239, 1986). Washing to remove unbound material, and the release of the bound cells, are performed using conventional methods.

### Measuring Activity

Polypeptides also find use in measuring the biological activity of the binding partner protein in terms of their binding affinity. The polypeptides thus may be employed by those conducting "quality assurance" studies, e.g., to monitor shelf life and stability of protein under different conditions. For example, the polypeptides may be employed in a binding affinity study to measure the biological activity of a binding partner protein that has been stored at different temperatures, or produced in different cell types. The proteins also may be used to determine whether biological activity is retained after modification of a binding partner protein (e.g., chemical modification, truncation, mutation, etc.). The binding affinity of the modified binding partner protein is compared to that of an unmodified binding partner protein to detect any adverse impact of the modifications on biological activity of the binding partner. The biological activity of a binding partner protein thus can be ascertained before it is used in a research study, for example.

### Delivery Agents

The polypeptides also find use as carriers for delivering agents attached thereto to cells bearing the binding partner. The polypeptides thus can be used to deliver diagnostic or therapeutic agents to such cells (or to other cell types found to express the binding partner on the cell surface) in *in vitro* or *in vivo* procedures.

Detectable (diagnostic) and therapeutic agents that may be attached to a polypeptide include, but are not limited to, toxins, other cytotoxic agents, drugs, radionuclides, chromophores, enzymes that catalyze a colorimetric or fluorometric reaction, and the like, with the particular agent being chosen according to the intended application. Among the toxins are ricin, abrin, diphtheria toxin, *Pseudomonas aeruginosa* exotoxin A, ribosomal inactivating proteins, mycotoxins such as trichothecenes, and derivatives and fragments (e.g., single chains) thereof. Radionuclides suitable for diagnostic use include, but are not limited to, ¹²³I, ¹³¹I, ^{99m}Tc, ¹¹¹In, and ⁷⁶Br. Examples of radionuclides suitable for therapeutic use are ¹³¹I, ²¹¹At, ⁷⁷Br, ¹⁸⁶Re, ¹⁸⁸Re, ²¹²Pb, ²¹²Bi, ¹⁰⁹Pd, ⁶⁴Cu, and ⁶⁷Cu.

Such agents may be attached to the polypeptide by any suitable conventional procedure. The polypeptide comprises functional groups on amino acid side chains that can be reacted with functional groups on a desired agent to form covalent bonds, for example. Alternatively, the protein or agent may be derivatized to generate or attach a desired reactive functional group. The derivatization may involve attachment of one of the bifunctional coupling reagents available for attaching various molecules to proteins (Pierce Chemical Company, Rockford, Illinois). A number of techniques for radiolabeling proteins are known. Radionuclide metals may be attached to polypeptides by using a suitable bifunctional chelating agent, for example.

Conjugates comprising polypeptides and a suitable diagnostic or therapeutic agent (preferably covalently linked) are thus prepared. The conjugates are administered or otherwise employed in an amount appropriate for the particular application.

### Therapeutic Agents

Polypeptides of the invention may be used in developing treatments for any disorder mediated (directly or indirectly) by defective, or insufficient amounts of the polypeptides. These polypeptides may be administered to a mammal afflicted with such a disorder.

The polypeptides disclosed herein may be employed in inhibiting a biological activity of the binding partner, in *in vitro* or *in vivo* procedures. For example, a purified Xrec2 receptor polypeptide may be used to inhibit binding of Xrec2 ligand to endogenous cell surface Xrec2 receptor, or a purified IL-1 zeta polypeptide, or any of its splice variants can be used to inhibit binding of endogenous IL-1 zeta or splice variants to its cell surface receptor. Biological effects that result from the binding of Xrec2 ligand to endogenous Xrec2 receptors thus are inhibited.

Polypeptides of the invention may be administered to a mammal to treat a binding partner-mediated disorder. Such binding partner-mediated disorders include conditions caused (directly or indirectly) or exacerbated by the binding partner.

Compositions of the present invention may contain a polypeptide in any form described herein, such as native proteins, variants, derivatives, oligomers, and biologically active fragments. In particular embodiments, the composition comprises a soluble polypeptide or an oligomer comprising soluble polypeptides of the invention.

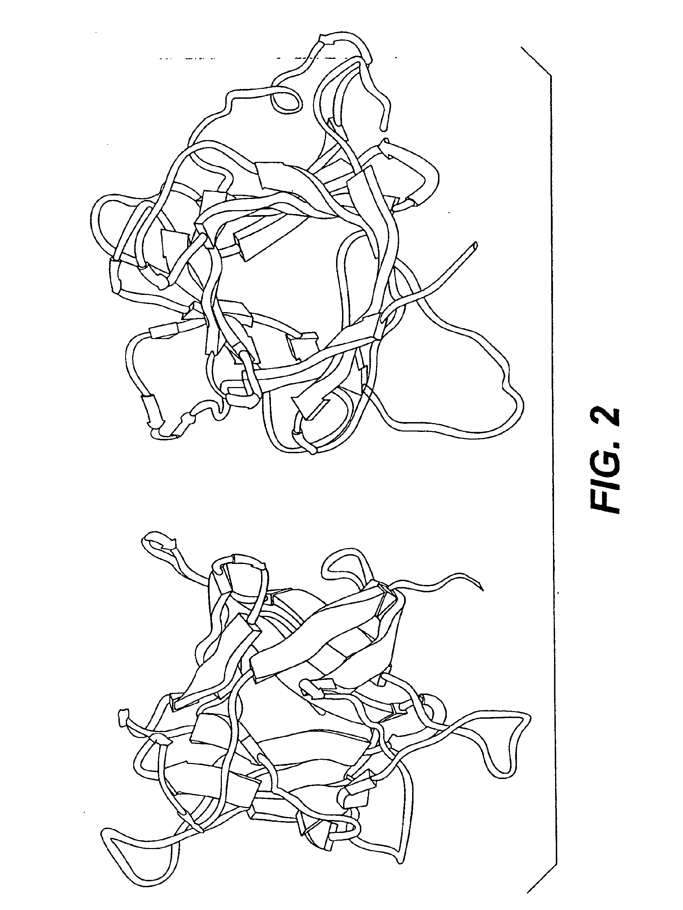

Particular regions of interest in the IL-1 zeta polypeptide may be derived from the molecular model provided in Figure 2, which depicts the secondary structure of the molecule. The model is based upon the crystal structures of IL-1 β and IL-1ra. In the figure, β-strands are shown in yellow, with their direction indicated by the arrowhead. β-turns are shown in blue, and coils are shown in green. The model demonstrates that it is possible to overlay the IL-1 zeta structure onto the IL-1β and IL-1ra structure without straining the molecule. The high degree of confidence in this molecular model allows it to be used in rational drug design to generate therapeutic molecules derived from IL-1 zeta.

Compositions comprising an effective amount of a polypeptide of the present invention, in combination with other components such as a physiologically acceptable diluent, carrier, or excipient, are provided herein. The polypeptides can be formulated according to known methods used to prepare pharmaceutically useful compositions. They can be combined in admixture, either as the sole active material or with other known active materials suitable for a given indication, with pharmaceutically acceptable diluents (e.g., saline, Tris-HCl, acetate, and phosphate buffered solutions), preservatives (e.g., thimerosal, benzyl alcohol, parabens), emulsifiers, solubilizers, adjuvants and/or carriers. Suitable formulations for pharmaceutical compositions include those described in Remington's Pharmaceutical Sciences, 16th ed., Mack Publishing Company, Easton, PA, 1980.

In addition, such compositions can be complexed with polyethylene glycol (PEG), metal ions, or incorporated into polymeric compounds such as polyacetic acid, polyglycolic acid, hydrogels, dextran, etc., or incorporated into liposomes, microemulsions, micelles, unilamellar or multilamellar vesicles, erythrocyte ghosts or spheroblasts. Such compositions will influence the physical state, solubility, stability, rate of *in vivo* release, and rate of *in vivo* clearance, and are thus chosen according to the intended application.

The compositions of the invention can be administered in any suitable manner, e.g., topically, parenterally, or by inhalation. The term "parenteral" includes injection, e.g., by subcutaneous, intravenous, or intramuscular routes, also including localized administration, e.g., at a site of disease or injury. Sustained release from implants is also contemplated. One skilled in the pertinent art will recognize that suitable dosages will vary, depending upon such factors as the nature of the disorder to be treated, the patient's body weight, age, and general condition, and the route of administration.

Preliminary doses can be determined according to animal tests, and the scaling of dosages for human administration is performed according to art-accepted practices.

Compositions comprising nucleic acids in physiologically acceptable formulations are also contemplated. DNA may be formulated for injection, for example.

### Research Agents

Another use of the polypeptides disclosed herein is as a research tool for studying the biological effects that result from the interactions of IL-1 zeta, or any of its splice variants, with its binding partner, and ofXrec2 with its binding partner, or from inhibiting these interactions, on different cell types. Polypeptides also may be employed in *in vitro* assays for detecting IL-1 zeta, Xrec2, the respective binding partners or the interactions thereof.

The polypeptides disclosed herein can be used to study cell signal transduction. IL-1 family ligands and receptors play a central role in protection against infection and immune inflammatory responses which includes cellular signal transduction, activating vascular endothelial cells and lymphocytes, induction of inflammatory cytokines, acute phase proteins, hematopoiesis, fever, bone resorption, prostaglandins, metalloproteinases, and adhesion molecules. With the continued increase in the number of known IL-1 family members, a suitable classification scheme is one based on comparing polypeptide structure as well as function (activation and regulatory properties). Thus, IL-1 zeta, TDZ.1, TDZ.2, and TDZ.3, like other IL-1 family ligands (IL-1α, IL-1β, and IL=18) and Xrec2, like other IL-1R family receptors (IL-1RI, IL-1RII, IL-1Rrp1, and AcPL), would likely be involved in many of the functions noted above as well as promote inflammatory responses and therefore perhaps be involved in the causation and maintenance of inflammatory and/or autoimmune diseases such as rheumatoid arthritis, inflammatory bowel disease, and psoriasis. As such, alterations in the expression and/or activation of the polypeptides disclosed herein can have profound effects on a plethora of cellular processes, including, but not limited to, activation or inhibition of cell specific responses and proliferation. Expression of cloned IL-1 zeta, TDZ.1, TDZ.2, TDZ.3, Xrec2, or of functionally inactive mutants thereof can be used to identify the role a particular protein plays in mediating specific signaling events.

Cellular signaling often involves a molecular activation cascade, during which a receptor propagates a ligand-receptor mediated signal by specifically activating intracellular kinases which phosphorylate target substrates. These substrates can themselves be kinases which become activated following phosphorylation. Alternatively, they can be adaptor molecules that facilitate down stream signaling through protein-protein interaction following phosphorylation. Regardless of the nature of the substrate molecule(s), expressed functionally active versions of Xrec2, IL-1 zeta, IL-1 zeta splice variants, and their binding partners can be used to identify what substrate(s) were recognized and activated by the polypeptides of the invention. As such, these novel polypeptides can be used as reagents to identify novel molecules involved in signal transduction pathways.

### Identification of Unknown Proteins

A polypeptide or peptide fingerprint can be entered into or compared to a database of known proteins to assist in the identification of the unknown protein using mass spectrometry (W.J. Henzel et al., *Proc. Natl. Acad. Sci. USA,* 90:5011-5015, 1993; Fenyo et al., *Electrophoresis,* 19:998-1005, 1998). A variety of computer software programs to facilitate these comparisons are accessible via the Internet, such as Protein Prospector (Internet site: prospector.uscf.edu), MultiIdent (Internet site: www.expasy.ch/sprot/multiident.html), PeptideSearch (Internet site:www.mann.embl-heiedelberg.de...deSearch/FR_PeptideSearch Form.html), and ProFound (Internet site:www.chait-sgi.rockefeller.edu/cgi-bin/ prot-id-frag.html). These programs allow the user to specify the cleavage agent and the molecular weights of the fragmented peptides within a designated tolerance. The programs compare observed molecular weights to predicted peptide molecular weights derived from sequence databases to assist in determining the identity of the unknown protein.

In addition, a polypeptide or peptide digest can be sequenced using tandem mass spectrometry (MS/MS) and the resulting sequence searched against databases (Eng et al., *J. Am. Soc. Mass Spec.,* 5:976-989, 1994; M. Mann et al., *Anal. Chem.,* 66:4390-4399, 1994; and J.A. Taylor et al., *Rapid Comm. Mass Spec.,* 11:1067-1075, 1997). Searching programs that can be used in this process exist on the Internet, such as Lutefisk 97 (Internet site: www.lsbc.com:70/Lutefisk97.html), and the Protein Prospector, Peptide Search and ProFound programs described above.

Therefore, adding the sequence of a gene and its predicted protein sequence and peptide fragments to a sequence database can aid in the identification of unknown proteins using mass spectrometry.

### Antibodies

Antibodies that are immunoreactive with the polypeptides of the invention are provided herein. Such antibodies specifically bind to the polypeptides *via* the antigen-binding sites of the antibody (as opposed to non-specific binding). Thus, the polypeptides, fragments, variants, fusion proteins, etc., as set forth above may be employed as "immunogens" in producing antibodies immunoreactive therewith. More specifically, the polypeptides, fragment, variants, fusion proteins, etc. contain antigenic determinants or epitopes that elicit the formation of antibodies.

These antigenic determinants or epitopes can be either linear or conformational (discontinuous). Linear epitopes are composed of a single section of amino acids of the polypeptide, while conformational or discontinuous epitopes are composed of amino acids sections from different regions of the polypeptide chain that are brought into close proximity upon protein folding (C. A. Janeway, Jr. and P. Travers, Immuno Biology. 3:9, Garland Publishing Inc., 2nd ed., 1996). Because folded proteins have complex surfaces, the number of epitopes available is quite numerous; however, due to the conformation of the protein and steric hinderances, the number of antibodies that actually bind to the epitopes is less than the number of available epitopes (C. A. Janeway, Jr. and P. Travers, Immuno Biology 2:14, Garland Publishing Inc., 2nd ed., 1996). Epitopes may be identified by any of the methods known in the art.

Antigenic epitopes of the polypeptides of the invention are useful for raising antibodies, in particular monoclonal antibodies, as described in more detail below. Additionally, epitopes from the polypeptides of the invention can be used as research reagents, in assays, and to purify specific binding antibodies from substances such as polyclonal sera or supernatants from cultured hybridomas. Such epitopes or variants thereof can be produced using techniques well known in the art such as solid-phase synthesis, chemical or enzymatic cleavage of a polypeptide, or using recombinant DNA technology.

As to the antibodies that can be elicited by the epitopes of the polypeptides of the invention, whether the epitopes have been isolated or remain part of the polypeptides, both polyclonal and monoclonal antibodies may be prepared by conventional techniques. See, for example, Kennet et al. (eds.), Monoclonal Antibodies, Hybridomas: A New Dimension in Biological Analyses, Plenum Press, New York, 1980; and Harlow and Land (eds.), Antibodies: A Laboratory Manual, Cold Spring Harbor Laboratory Press, Cold Spring Harbor, NY, 1988.

Hybridoma cell lines that produce monoclonal antibodies specific for the polypeptides of the invention are also contemplated herein. Such hybridomas may be produced and identified by conventional techniques. One method for producing such a hybridoma cell line comprises immunizing an animal with a polypeptide; harvesting spleen cells from the immunized animal; fusing said spleen cells to a myeloma cell line, thereby generating hybridoma cells; and identifying a hybridoma cell line that produces a monoclonal antibody that binds the polypeptide. The monoclonal antibodies may be recovered by conventional techniques.

The monoclonal antibodies of the present invention include chimeric antibodies, e.g., humanized versions of murine monoclonal antibodies. Such humanized antibodies may be prepared by known techniques and offer the advantage of reduced immunogenicity when the antibodies are administered to humans. In one embodiment, a humanized monoclonal antibody comprises the variable region of a murine antibody (or just the antigen binding site thereof) and a constant region derived from a human antibody. Alternatively, a humanized antibody fragment may comprise the antigen binding site of a murine monoclonal antibody and a variable region fragment (lacking the antigen-binding site) derived from a human antibody. Procedures for the production of chimeric and further engineered monoclonal antibodies include those described in Riechmann et al., *Nature,* 332:323, 1988; Liu et al., *PNAS,* 84:3439, 1987; Larrick et al., *Bio*/*Technology,* 7:934, 1989; and Winter et al., *TIPS,* 14:139, 1993. Procedures to generate antibodies transgenically can be found in GB 2,272,440, US Patent Nos. 5,569,825 and 5,545,806 and related patents claiming priority therefrom.

Antigen-binding fragments of the antibodies, which may be produced by conventional techniques, are also encompassed by the present invention. Examples of such fragments include, but are not limited to, Fab and F(ab')₂ fragments. Antibody fragments and derivatives produced by genetic engineering techniques are also provided.

In one embodiment, the antibodies are specific for the polypeptides of the present invention and do not cross-react with other proteins. Screening procedures by which such antibodies may be identified are well known, and may involve immunoaffinity chromatography, for example.

### Uses Thereof

The antibodies of the invention can be used in assays to detect the presence of the polypeptides or fragments of the invention, either *in vitro* or *in vivo.* The antibodies also may be employed in purifying polypeptides or fragments of the invention by immunoaffinity chromatography.

Those antibodies that additionally can block binding of the polypeptides of the invention to the binding partner may be used to inhibit a biological activity that results from such binding. Such blocking antibodies may be identified using any suitable assay procedure, such as by testing antibodies for the ability to inhibit binding of IL-1 zeta, TDZ.1 or TDZ.2 to certain cells expressing the IL-1 zeta receptors. Alternatively, blocking antibodies may be identified in assays for the ability to inhibit a biological effect that results from polypeptides of the invention binding to their binding partners to target cells. Antibodies may be assayed for the ability to inhibit IL-1 zeta-mediated, or binding partner-mediated cell lysis, for example.

Such an antibody may be employed in an *in vitro* procedure, or administered *in vivo* to inhibit a biological activity mediated by the entity that generated the antibody. Disorders caused or exacerbated (directly or indirectly) by the interaction of the polypeptides of the invention with the binding partner thus may be treated. A therapeutic method involves *in vivo* administration of a blocking antibody to a mammal in an amount effective in inhibiting a binding partner-mediated biological activity. Monoclonal antibodies are generally preferred for use in such therapeutic methods. In one embodiment, an antigen-binding antibody fragment is employed.

Antibodies may be screened for agonistic (*i.e*., ligand-mimicking) properties. Such antibodies, upon binding to cell surface receptor, induce biological effects (e.g., transduction of biological signals) similar to the biological effects induced when IL-1 binds to cell surface IL-1 receptors. Agonistic antibodies may be used to activate vascular endothelial cells and lymphocytes, induce local tissue destruction and fever (Janeway et al., 1996), stimulate macrophages and vascular endothelial cells to produce IL-6, and upregulate molecules on the surface of vascular endothelial cells.

Compositions comprising an antibody that is directed against polypeptides of the invention, and a physiologically acceptable diluent, excipient, or carrier, are provided herein. Suitable components of such compositions are as described above for compositions containing polypeptides of the invention.

Also provided herein are conjugates comprising a detectable (e.g., diagnostic) or therapeutic agent, attached to the antibody. Examples of such agents are presented above. The conjugates find use in *in vitro* or *in vivo* procedures.

The following examples are provided to further illustrate particular embodiments of the invention, and are not to be construed as limiting the scope of the present invention. It is to be noted that TDZ.3 and Xrec2 is not fall within the scope of the present invention.

### EXAMPLE 1: Isolation of the IL-1 zeta and Xrec2 Nucleic Acids

Human IL-1 zeta nucleic acid sequence was obtained by sequencing EST IMAGE clone 1628761, accession #AI014548, which encodes a partial open reading frame (ORF). A number of cDNA libraries were screened with internal primers to determine the expression pattern of the polypeptide. After performing PCR using two internal primers of human IL-1 zeta sequence, the following cDNA libraries were positive for IL-1 zeta sequences: bone marrow stromal, human pancreatic tumor, and Raji (B-cell line). IL-1 zeta clones were isolated from human genomic DNA sequences, bone marrow stromal and human pancreatic tumor libraries, and sequenced.

Human Xrec2 sequences were obtained by high-throughput sequencing, PCR, and 5' RACE reactions. High-throughput shotgun sequencing of chromosome region Xp11 yielded sequences for exons 4-6 of Xrec2 (Genbank accession numbers AL031466 and AL031575). Similarly, sequence of chromosome region Xp22-164-166 (Genbank accession number AC005748) yielded sequences for exons 10 - 12 of Xrec2.

PCR (40 cycles) was performed on human brain first strand cDNA using primers (10 picomoles/reaction) within exons 5 and 11 and Hotstar Taq polymerase (Qiagen, Valencia, CA), generating sequence for exons 7-9. 5' RACE reactions were then performed using testis cDNA and nested primers within exon 4 to obtain exon 3 sequences which contained the predicted initiator methionine. Both PCR and the 5' RACE reactions were performed using standard protocols.

### EXAMPLE 2: Use of Purified IL-1 zeta and Xrec2 Polypeptides

### Polypeptide-specific ELISA:

Serial dilutions of IL-1 zeta- or Xrec2-containing samples (in 50 mM NaHCO₃, brought to pH 9 with NaOH) are coated onto Linbro/Titertek 96 well flat bottom E.LA. microtitration plates (ICN Biomedicals Inc., Aurora, OH) at 100:1/well. After incubation at 4 C for 16 hours, the wells are washed six times with 200:1 PBS containing 0.05% Tween-20 (PBS-Tween). The wells are then incubated with FLAG® -binding partner at 1 mg/ml in PBS-Tween with 5% fetal calf serum (FCS) for 90 minutes (100:1 per well), followed by washing as above. Next, each well is incubated with the anti-FLAG® (monoclonal antibody M2 at 1 mg/ml in PBS-Tween containing 5% FCS for 90 minutes (100:1 per well), followed by washing as above. Subsequently, wells are incubated with a polyclonal goat anti-mIgG1-specific horseradish peroxidase-conjugated antibody (a 1:5000 dilution of the commercial stock in PBS-Tween containing 5% FCS) for 90 minutes (100 :1 per well). The HRP-conjugated antibody is obtained from Southern Biotechnology Associates, Inc., Birmingham, Alabama. Wells then are washed six times, as above.

For development of the ELISA, a substrate mix [100:1 per well of a 1:1 premix of the TMB Peroxidase Substrate and Peroxidase Solution B (Kirkegaard Perry Laboratories, Gaithersburg, Maryland)] is added to the wells. After sufficient color reaction, the enzymatic reaction is terminated by addition of 2 N H₂SO₄ (50:1 per well). Color intensity (indicating ligand receptor binding) is determined by measuring extinction at 450 nm on a V Max plate reader (Molecular Devices, Sunnyvale, CA).

### EXAMPLE 3: Amino Acid Sequence

The amino acid sequence of IL-1 zeta and Xrec2 were determined by translation of the complete nucleotide sequences of SEQ ID NOs:1 and 2, respectively.

### EXAMPLE 4: DNA and Amino Acid Sequences

The IL-1 zeta and Xrec2 nucleic acid sequences were determined by standard double stranded sequencing of the composite sequence of EST IMAGE clones (accession #AI014548 (IL-1 zeta) and # AL031575 and #AC005748 (Xrec2)), and of additional sequences obtained from PCR and 5' RACE reactions.

The nucleotide sequence of the isolated IL-1 zeta and Xrec2 DNA and the amino acid sequence encoded thereby, are presented in SEQ ID NOs:1-4. The sequence of the IL-1 zeta DNA fragment isolated by PCR corresponds to nucleotides 1 to 579 of SEQ ID NO:1. which encode amino acids 1 to 192 of SEQ ID NO:3; and the sequence of the Xrec2 DNA fragment also isolated by PCR corresponds to nucleotides 1 to 2088 of SEQ ID NO:2, which encode amino acids 1 to 698 of SEQ ID NO:4.

The amino acid sequences of SEQ ID NOs:3 and 4 bear significant homology to other known IL-1 ligand and receptor family members, respectively.

### EXAMPLE 5: Monoclonal Antibodies That Bind Polypeptides of the Invention

This example illustrates a method for preparing monoclonal antibodies that bind IL-1 zeta polypeptide. The same protocol can be used to produce monoclonal antibodies that bind Xrec2 polypeptide. Suitable immunogens that may be employed in generating such antibodies include, but are not limited to, purified IL-1 zeta polypeptide or an immunogenic fragment thereof such as the extracellular domain, or fusion-proteins containing IL-1-zeta (e.g., a-soluble IL-1 zeta/Fc fusion protein).

Purified IL-1 zeta polypeptide can be used to generate monoclonal antibodies immunoreactive therewith, using conventional techniques such as those described in U.S. Patent 4,411,993. Briefly, mice are immunized with IL-1 zeta immunogen emulsified in complete Freund's adjuvant, and injected in amounts ranging from 10-100 µg subcutaneously or intraperitoneally. Ten to twelve days later, the immunized animals are boosted with additional IL-1 zeta emulsified in incomplete Freund's adjuvant. Mice are periodically boosted thereafter on a weekly to bi-weekly immunization schedule. Serum samples are periodically taken by retro-orbital bleeding or tail-tip excision to test for IL-1 zeta antibodies by dot blot assay, ELISA (Enzyme-Linked Immunosorbent Assay) or inhibition of IL-1 zeta receptor binding. Following detection of an appropriate antibody titer, positive animals are provided one last intravenous injection of IL-1 zeta in saline. Three to four days later, the animals are sacrificed, spleen cells harvested, and spleen cells are fused to a murine myeloma cell line, e.g., NS1 or preferably P3x63Ag8.653 (ATCC CRL 1580). Fusions generate hybridoma cells, which are plated in multiple microtiter plates in a HAT (hypoxanthine, aminopterin and thymidine) selective medium to inhibit proliferation of non-fused cells, myeloma hybrids, and spleen cell hybrids.

The hybridoma cells are screened by ELISA for reactivity against purified IL-1 zeta by adaptations of the techniques disclosed in Engvall et al., *Immunochem.,* 8:871, 1971 and in U.S. Patent 4,703,004. A preferred screening technique is the antibody capture technique described in Beckmann et al., *J. Immunol.,* 144:4212, 1990. Positive hybridoma cells can be injected intraperitoneally into syngeneic BALB/c mice to produce ascites containing high concentrations of anti-IL-1 zeta monoclonal antibodies. Alternatively, hybridoma cells can be grown *in vitro* in flasks or roller bottles by various techniques. Monoclonal antibodies produced in mouse ascites can be purified by ammonium sulfate precipitation, followed by gel exclusion chromatography. Alternatively, affinity chromatography based upon binding of antibody to Protein A or Protein G can also be used, as can affinity chromatography based upon binding to IL-1 zeta.

### EXAMPLE 6: Tissue Distribution of Xrec2 mRNA

The tissue distribution of Xrec2 mRNA was investigated by Northern blot analysis, as follows. An aliquot of an Xrec2 riboprobe was added to two different multiple human tissue Northern blots (Clontech, Palo Alto, CA; Biochain, Palo Alto, CA). The blots were hybridized in 10X Denhardt's solution, 50mM Tris pH 7.5, 900mM NaCl, 0.1% Na pyrophosphate, 1% SDS, 200µg/mL salmon sperm DNA. Hybridization was conducted overnight at 63 °C in 50% formamide as previously described (March et al., *Nature* 315:641-647, 1985). The blots then were washed with 2X SSC, 0.1% SDS at 68°C for 30 minutes. The cells and tissues with the highest levels of Xrec2 mRNA were determined by comparison to control probing with a β-actin-specific probe.

Xrec2 was detected in human brain and heart tissues, and to a lesser extent in ovary. In these tissues, two Xrec2 mRNAs were detected, one 7.5 kb transcript and one 10.0 kb transcript. An 8.0 kb Xrec2 transcript was detected in skeletal muscle. PCR analysis of a human cDNA tissue panel detected Xrec2 mRNA in heart, brain, and ovary, and to a lesser extent in tonsil, fetal liver, prostate, testis, small intestine and colon but not in spleen, lymph node, thymus, bone marrow, leukocytes, placenta, lung, liver, skeletal muscle, kidney or pancreas.

Following the procedures described above, a Northern blot of RNA isolated from tumor cells was probed with Xrec2. An 8.0 kb transcript that hybridized weakly to the probe was detected in SW480, a colorectal adenocarincoma cell line. Xrec2 transcripts were not detected in HL-60 (promyeloctyic leukemia), S3 (HeLa cell), K-562 (chronic myelogenous leukemia), MOLT-4 (lymphoblastic leukemia), Raji (Burkitt lymphoma), A5 49 (lung carcinoma), or G361 (melanoma) cells.

### EXAMPLE 7: Binding Assay for IL-1 Zeta

Full length IL-1 zeta can be expressed and tested for the ability to bind IL-1 zeta receptors. The binding assay can be conducted as follows.

A fusion protein comprising a leucine zipper peptide fused to the N-terminus of a soluble IL-1 zeta polypeptide (LZ-IL-1 zeta) is employed in the assay. An expression construct is prepared, essentially as described for preparation of the -FLAG® (IL-1 zeta) expression construct in Wiley et al., *Immunity,* 3:673-682, 1995, hereby incorporated by reference, except that DNA encoding the FLAG® peptide was replaced with a sequence encoding a modified leucine zipper that allows for trimerization. The construct, in expression vector pDC409, encodes a leader sequence derived from human cytomegalovirus, followed by the leucine zipper moiety fused to the N-terminus of a soluble IL-1 zeta polypeptide. The LZ-IL-1 zeta is expressed in CHO cells, and purified from the culture supernatant.

The expression vector designated pDC409 is a mammalian expression vector derived from the pDC406 vector described in McMahan et al. *EMBO J.,* 10:2821-2832, 1991, hereby incorporated by reference. Features added to pDC409 (compared to pDC406) include additional unique restriction sites in the multiple cloning site (mcs); three stop codons (one in each reading frame) positioned downstream of the mcs; and a T7 polymerase promoter, downstream of the mcs, that facilitates sequencing of DNA inserted into the mcs.

For expression of full length human IL-1 zeta protein, the entire coding region *(i.e.,* the DNA sequence presented in SEQ ID NO:1) is amplified by polymerase chain reaction (PCR). The template employed in the PCR is the cDNA clone isolated from a (pancreatic tumor) cDNA library, as described in example 1. The isolated and amplified DNA is inserted into the expression vector pDC409, to yield a construct designated pDC409-IL-1 zeta.

LZ-IL-1 zeta polypeptide is employed to test the ability to bind to host cells expressing recombinant or endogenous IL-1 zeta receptors, as discussed above. Cells expressing IL-1 zeta receptor are cultured in DMEM supplemented with 10% fetal bovine serum, penicillin, streptomycin, and glutamine. Cells are incubated with LZ-IL-1 zeta (5 mg/ml) for about 1 hour. Following incubation, the cells are washed to remove unbound LZ-IL-1 zeta and incubated with a biotinylated anti-LZ monoclonal antibody (5 mg/ml), and phycoerythrin-conjugated streptavidin (1:400), before analysis by fluorescence-activated cell scanning (FACS). The cytometric analysis is conducted on a FACscan (Beckton Dickinson, San Jose, CA).

The cells expressing IL-1 zeta receptors will show significantly enhanced binding of IL-1=zeta, compared to the control cells not expressing IL-1 zeta receptors.

### EXAMPLE 8: Identification And Tissue Distribution of IL-1 Zeta and its Splice Variants, TDZ.1, TDZ.2, and TDZ.3

Expression of IL-1 zeta in various human tissues was examined by RT PCR with IL-1 zeta specific primers or by screening cDNA libraries with a radiolabeled DNA probe derived from the IL-1 zeta EST described in Example 1. The results are shown in Table II.

In Table II, "-" indicates that in these experiments IL-1 zeta mRNA was not detected in the particular tissue or cell line analyzed. Given the limitations of the assays used, it will be recognized that "-" indicates only that IL-1 zeta was not detected in a particular tissue in a particular experiment and does not imply that IL-1 zeta is never expressed in that tissue. Furthermore, varying expression patterns could be explained by different lots of RNA source material being used to generate the cDNA tissue panels.

Positive results were derived as follows: "a", by PCR analysis from a panel of first strand cDNAs (Clontech); "b", by cDNA library screening; "c", by the existence of an EST; and "d", by PCR analysis of an individual RNA. "e" indicates that expression of the gene was increased by addition of LPS to the cells. In the source column for tissues, "Pool" was a mixture of fetal lung, testis and B cells. In the source column for human cell lines, "macrophage-1" was THP-1, "macrophage-2" was U937; "BM stroma" was an unpublished bone marrow stromal cell line, IMTLH, derived at Immunex; "early hemat." was the hematopoietic precursor line HL60; and "panc. tumor" was HPT-4.

As shown in the Table, IL-1 zeta mRNA was detected in human lymph node, thymus, bone marrow stroma, lung, testis and placenta. In addition, IL-1 zeta mRNA was detected in human macrophage cell lines THP-1 and U937, hematopoietic precursor cell line HL60, and pancreatic tumor cell line HPT-4.

**TABLE II**

| **SOURCE** | **IL-1 ZETA** |
|---|---|
| **Human Tissue** | |
| Spleen | - |
| Lymph Node | a |
| Thymus | a |
| Tonsil | |
| Bone Marrow | a, b |
| Fetal Liver | |
| Leukocyte | |
| Heart | |
| Placenta | a |
| Lung | a |
| Liver | - |
| Skeletal Muscle | - |
| Kidney | - |
| Pancreas | - |
| Prostate | - |
| Testis | a |
| Ovary | |
| Small Intestine | |
| Colon | |
| Colon tumor | c |
| Pool | c |

| **Human cell lines** | |
|---|---|
| macrophage-1 | d |
| macrophage-2 | d, e |
| BM stroma | d |
| Early hemat. | d |
| Panc. tumor | b |

The tissue distribution of IL-1 zeta was also compared to the tissue distribution of Tango-77 (WO 99/06426), an alternatively spliced form of IL-1 zeta, using RT PCR. The primers used in the RT PCR were 5' primers specific for either the first exon of Tango-77-(exon (1) in Figure 1) or the first exon of IL-1 zeta (exon (3) in Figure 1) in combination with a common 3' primer derived from the common terminal exon (exon (6) in Figure 1). The PCR reactions were performed using first strand cDNA from multiple human tissue sources purchased from Clontech, Palo Alto, CA. PCR products of the predicted size as well as several additional PCR products of a different size were detected. Three PCR products of different sizes than the predicted size were isolated and used to obtain sequence information from multiple tissue cDNAs. Isolation and characterization of these PCR products revealed three novel IL-1 zeta splice variants. The nucleic acid sequences of these splice variants are represented by SEQ ID NO: 5, 6, and 7, which encode the amino acid sequences represented by SEQ ID NOS: 8, 9, and 10, respectively. The organization and relationship of these splice variants is shown in Figure 1.

The splice variants were designated TDZ.1, TDZ.2, and TDZ.3 (Testis-Derived Zeta variants) because they are each expressed in testis. Testis is a common expression tissue, however, it is not the only expression tissue. Table III shows the results of a tissue expression study for IL-1 zeta, Tango-77, TDZ.1, TDZ.2, and TDZ.3. TDZ.1 and TDZ.2 contain exons 4, 5, and 6, shown in Figure 1, which correspond to the last three exons of IL-1 zeta and correspond to the conserved structural domain of the molecule. When aligned with other members of the IL-1 family, exons 4, 5, and 6 are shown to contain many conserved residues within conserved structural motifs.

A polymorphism of Tango 77 in exon (2) of Figure 1 is noted. In the isolated cDNAs a valine occurs in lieu of a glycine at the third residue of exon (2). In the Tango-77 sequence, the amino acid sequence is PAGSPLEP (SEQ ID NO: 14). In the polymorphism the sequence is PAVSPLEP (SEQ ID NO: 15).

**TABLE III TISSUE DISTRIBUTION OF IL-1 ZETA AND IL-1 ZETA SPLICE VARIANTS**

| **Tissue** | **IL-1z** | **Tango-77** | **TDZ.1** | **TDZ.2** | **TDZ.3** |
|---|---|---|---|---|---|
| kidney | - | - | + | - | - |
| pancreas | - | - | - | - | - |
| skeletal muscle | - | - | + | - | - |
| heart | - | + | - | - | - |
| testis | + | + | + | + | + |
| prostate | + | - | + | - | - |
| spleen | - | - | - | - | - |
| ovary | - | + | + | - | - |
| thymus | - | - | - | - | - |
| colon | + | + | + | - | - |
| leukocytes | - | - | - | - | - |
| small intestine | - | + | + | - | - |
| liver | - | + | + | - | - |
| brain | + | - | - | - | - |
| placenta | + | + | + | - | + |
| lung | + | + | + | - | + |
| tonsil | - | + | + | - | - |
| fetal liver | + | + | + | - | - |
| lymph node | + | + | + | - | - |
| bone marrow | - | + | + | + | + |

### EXAMPLE 9: Signaling Activity of Xrec2

Full length Xrec2 was cloned into the expression vector pDC304. The resultant vector and an NFκB-driven luciferase reporter plasmid were used to transfect COS7 cells by the DEAE-Dextran method as described previously in Born et al., *J. Biol. Chem.,* 273: 29445-29450, 1998, hereby incorporated by reference. When the transfected cells were stimulated for 4 hours with IL-1α (10 ng/ml), IL-1β (10 ng/ml), or hIL-18 (40 ng/ml), no luciferase activity was detected.

Several known members of the IL-1R family are orphan receptors for which no known cognate ligands have yet been identified. Examples of such orphan receptors include IL-1Rrp2, T1/ST2 and SIGIRR. Several of these orphan receptors can, however, mediate transcriptional activation in response to IL-1, when expressed as chimeric molecules with the IL-1R extracellular and transmembrane domains (IL-1Rextm) fused to the cytoplasmic domain of the orphan receptor. For example, an IL-1Rextm chimera containing the cytoplasmic domain of T 1 /ST2 is able to mediate transcriptional activation in response to IL-1 stimulation, as outlined in Mitcham et al., *J. Biol. Chem.,* 271: 5777-5783, 1996, hereby incorporated by reference.

To test the ability of Xrec2 to mediate transcriptional activation in response to IL-1, the cytoplasmic domain of Xrec2 (Xrec2cyto) (amino acids 382-696 of SEQ ID NO:4) was expressed as a chimeric molecule with IL-1Rextm. The chimeric IL-1 Rextm-Xrec2cyto was overexpressed in COS7 cells together with an NFκB-driven luciferase reporter plasmid, as described above. Following IL-1 stimulation of these cells, no luciferase activity was detected. As a control, full length IL-1R was overexpressed in COS7 cells, and induction of transcriptional activity in response to IL-1 stimulation in these cells was observed.

The experiment was repeated using an IL-1Rextm-Xrec2cyto chimeric receptor with a truncated cytoplasmic tail (amino acids 382-573 of SEQ ID NO:4). Again, the chimeric receptor was non-responsive to IL-1 in these assays, indicating that unlike some other members of the IL-IR family, the cytoplasmic domain of Xrec2 does not mediate signal transduction through NFκB.

Other members of the IL-1 receptor family function as accessory subunits, similar to the IL-1 R AcP, which is a required signaling component of the type I IL-1R. To assess whether Xrec2 functions as an accessory subunit of the IL-1 receptor, a series of chimeric receptors were created. The cytoplasmic domain of each identified prototypical IL-1R family member was fused to the extracellular and transmembrane domains of both IL-1R and AcP, resulting in a panel of seven IL-1R chimeras and seven AcP chimeras. The IL-1R and AcP chimeras were co-expressed in all possible combinations in a murine T cell lymphoma cell line (S49.1) by electroporation, along with an NFκB-driven luciferase reporter plasmid. Two days after electroporation, the cells were stimulated with IL-1α (10 ng/ml) or IL-1 β (10 ng/ml) for 4 hours, and luciferase activity was assessed, as described previously in Bom et al., *J Biol. Chem.,* 273: 29445-29450, 1998. Normally, S49.1 cells are non-responsive to IL-1. However, upon transient overexpression of both IL-1R and AcP, S49.1 cells become IL-1 responsive. While other AcP-like molecules cooperated in this system with other IL-1R-like molecules to confer IL-1 responsiveness to the 549.1 cells, Xrec2 did not do so as either an IL-1R or an AcP chimera.

## Claims

1. An isolated polynucleotide comprising a nucleic acid molecule that encodes a polypeptide selected from the group consisting of
(a) a polypeptide comprising SEQ ID NO: 3;
(b) a polypeptide comprising SEQ ID NO: 8;
(c) a polypeptide comprising SEQ ID NO: 9;
(d) a polypeptide that is at least 95% identical to a polypeptide of a) or c), wherein the polypeptide binds an IL-1R family member; and
(e) a fragment of the polypeptide of SEQ ID NO: 3 whose N-terminal amino acid is selected from amino acids 21-51 and whose C-terminal amino acid is selected from amino acids 188-192 and which binds an IL-1R family member, wherein the fragment is not amino acids 24-192, 28-192 or 34-192 of SEQ ID NO: 3;
(f) a fragment of the polypeptide of SEQ ID NO: 8 whose N-terminal amino acid is selected from amino acids 41-61 and whose C-terminal amino acid is selected from amino acids 214-218 and which binds an IL-1R family member, wherein the fragment is not amino acids 43-218, 54-218 or 60-218 of SEQ ID NO: 8; and
(g) a fragment of the polypeptide of SEQ ID NO: 9 whose N-terminal amino acid is selected from amino acids 21-41 and whose C-terminal amino acid is selected from amino acids 193-197 and which binds an IL-1R family member, wherein the fragment is not amino acids 33-197 or 39-197 of SEQ ID NO: 9.

2. An isolated polynucleotide as claimed in claim 1, wherein the nucleic acid molecule has the sequence of SEQ ID NO: 1 or SEQ ID NO: 6.

3. An isolated polynucleotide as claimed in claim 1, wherein the N-terminal amino acid of the fragment of the polypeptide of SEQ ID NO: 8 is amino acid 52.

4. An isolated polynucleotide as claimed in claim 1, wherein the N-terminal amino acid of the fragment of the polypeptide of SEQ ID NO: 9 is amino acid 31.

5. An isolated polypeptide comprising a polypeptide selected from the group consisting of
(a) a polypeptide comprising SEQ ID NO: 3;
(b) a polypeptide comprising SEQ ID NO: 8;
(c) a polypeptide comprising SEQ ID NO: 9;
(d) a polypeptide that is at least 95% identical to a polypeptide of a)-c), wherein the polypeptide binds an IL-1 R family member,
(e) a fragment of the polypeptide of SEQ ID NO: 3 whose N-terminal amino acid is selected from amino acids 21-51 and whose C-terminal amino acid is selected from amino acids 188-192 and which binds an IL-IR family member, wherein the fragment is not amino acids 24-192, 28-192 or 34-192 of SEQ ID NO: 3;
(f) a fragment of the polypeptide of SEQ ID NO: 8 whose N-terminal amino acid is selected from amino acids 41-61 and whose C-terminal amino acid is selected from amino acids 214-218 and which binds an IL-1R family member, wherein the fragment is not amino acids 43-218, 54-218 or 60-218 of SEQ ID NO: 8; and
(g) a fragment of the polypeptide of SEQ ID NO: 9 whose N-terminal amino acid is selected from-amino acids 21-41 and whose C-terminal amino acid is selected from amino acids 193-197 and which binds an IL-1R family member, wherein the fragment is not amino acids 33-197 or 39-197 of SEQ ID NO: 9.

6. An isolated polypeptide as claimed in claim 5, wherein the N-terminal amino acid of the fragment of the polypeptide of SEQ ID NO: 8 is amino acid 52.

7. An isolated polypeptide as claimed in claim 5, wherein the N-terminal amino acid of the fragment of the polypeptide of SEQ ID NO: 9 is amino acid 31.

8. An expression vector comprising a polynucleotide according to any one of claim 1 to 4 claim.

9. A host cell transformed or transfected with the vector of claim 8.

10. A method for preparing a polypeptide, the method comprising culturing a host cell of claim 9 under conditions promoting expression of the polypeptide.

11. An oligomeric polypeptide comprising a polypeptide of claim 5, 6 or 7.

12. An antibody that binds to a polypeptide of claim 5, 6, 7 or 11.

13. The antibody of claim 12, wherein the antibody is a monoclonal antibody.

## Patentansprüche

1. Isoliertes Polynukleotid welches ein Nukleinsäuremolekül aufweist, dass ein Polypeptid kodiert, welches aus der Gruppe ausgewählt ist bestehend aus:
a) einem Polypeptid, welches die SEQ ID Nr. 3 anfweist;
b) einem Polypeptid, welches die SEQ ID Nr. 8 aufweist;
c) einem Polypeptid, welches die SEQ ID Nr. 9 aufweist;
d) einem Polypeptid das zumindest zu 95% identisch zu dem Polypeptid a) oder c) ist, wobei das Polypeptid an ein IL-1R Familienmitglied bindet; und
e) einem Fragment des Polypeptids der SEQ ID Nr. 3 dessen N-terminale Aminosäure aus den Aminosäuren 21-51 und dessen C-terminale Aminosäure aus den Aminosäuren 188 - 192 ausgewählt ist und welches an ein IL-1R Familienmitglied bindet, wobei das Fragment nicht die Aminosäuren 24 - 192, 28 - 192 oder 34 -192 der SEQ ID Nr. 3 sind;
f) einem Fragment des Polypeptides der SEQ ID Nr. 8 dessen. N-terminale Aminosäure aus den Aminosäuren 41 - 61 und dessen C-terminale Aminosäure aus den Aminosäuren 214 - 218 ausgewählt ist und welches an ein IL-IR Familienmitglied bindet, wobei das Fragment nicht die Aminosäuren 43 - 21.8, 54 - 218 oder 60 - 218 der SEQ ID Nr. 8 sind; und
g) einem Fragment des Polypeptides der SEQ ID Nr. 9 dessen, N-termmale Aminosäure aus den Aminosäuren 21 - 41 und dessen C-terminale Aminosäure aus den Aminosäuren 1.93 - 197 ausgewählt ist und welches an ein IL-1R Familienmitglied bündet, wobei das Fragment nicht die Aminosäuren 33 - 197 oder 39 -1.97 der SEQ ID Nr. 9 sind.

2. Isoliertes Polynukleotid nach Anspruch 1, wobei das Nukleinsäuremolekitle die SEQ ID Nr. 1 oder SEQ ED X-t. 6 hat.

3. Isoliertes Polynuklcolid nach Anspruch 1, wobei die N-terminale Aminosäure des Fragments des Polypeptides der SEQ ID Nr, 8 die Aminosäure 52 ist.

4. Isoliertes Polynukleotid nach Anspruch 1, wobei die N-terminale Aminosäure des Fragments des Polypeptids der SEQ ID Nr. 9 die Aminosäure 31 ist.

5. Isoliertes Polypeptid das ein Polypeptide anfweist, dass aus der Gruppe ausgewählt ist bestehend aus:
a) einem Polypeptid, welches die SEQ ID Nr. 3 aufWeist;
b) einem Polypeptid, welches die SEQ ID Nr. 8 aufweist;
c) einem Polypeptid, welches die SEQ ID Nr. 9 aufweist;
d) einem Polypeptid das zumindest zu 95% identisch zu dem Polypeptid a) oder o) ist, wobei das Polypeptid an ein IL-1R Familienmitglied bindet;
c) einem Fragment des Polypeptids der SEQ ID Nr. 3 dessen N-terminale Aminosäure aus den Aminosäuren 21-51 und dessen C-terminale Aminosäure aus den Aminosäuren 188 - 192 ausgewählt ist und welches an ein IL-1R Familienmitglied bindet, wobei das Fragment nicht die Aminosäuren 24 -192,28 -192 oder 34 -192 der SEQ ID Nr. 3 sind;
f) einem Fragment des Polypeptides der SEQ ID Nr. 8 dessen N-terminale Aminosäure aus den Aminosäuren 41 - 61 und dessen C-terminale Aminosäure aus den Aminosäuren 214 - 218 ausgewählt ist und welches an ein IL-1R Familienmitglied bindet, wobei das Fragment nicht die Aminosäuren 43 - 218, 54 - 218 oder 60 - 218 der SEQ ID Nr. 8 sind; und
g) einem Fragment des Polypeptides der SEQ ID Nr. 9 dessen N-terminale Aminosäure aus den Aminosäuren 21 - 41 und dessen C-terminale Aminosäure aus den Aminosäuren 193 - 197 ausgewählt ist und welches an ein IL-1R Familienmitglied bindet, wobei das Fragment nicht die Aminosäuren 33-197 oder 39-197 der SEQ ID Nr. 9 sind.

6. Isoliertes Polypeptid nach Anspruch 5, wobei die N-terminale Aminosäure des Fragments des Polypeptids der SEQ ID Nr. 8 die Aminosäure 52 ist.

7. Isoliertes Polypeptid nach Anspurch 5, wobei die N-terminale Aminosäure des Fragments des Polypeptids der SEQ ID Nr. 9 die Aminosäure 31 ist.

8. Expressjonsvektor der das Polypeptid nach einem der Ansprüche 1 bis 4 aufweist,

9. Wirtszelle die mit dem Vektor nach Anspruch 8 transformiert oder transfiziert ist.

10. Verfahren zum Herstellen eines Polypeptids, wobei das Verfahren das Kultivieren einer Wirtszelle nach Anspruch 9 unter Bedingungen die die Exprossion des Polypeptids fördern, aufweist.

11. Oligomeres Polypeptid, das ein Polypeptid nach Anspruch 5, 6 oder 7 aufweist.

12. Antikörper, welcher an das Polypeptid nach Anspruch 5, 6, 7 oder 11 bindet.

13. Antikörper nach Anspruch 12, wobei der Antikörper ein monoklonaler Antikörper ist.

## Revendications

1. Polynucléotide isolé comprenant une molécule d'acide nucléique qui code pour un polypeptide choisi dans le groupe constitué de :
(a) un polypeptide comprenant la SEQ ID NO :3 ;
(b) un polypeptide comprenant la SEQ ID NO :8 ;
(c) un polypeptide comprenant la SEQ ID NO :9 ;
(d) un polypeptide qui est identique au moins à 95% par rapport à un polypeptide de a) ou c), le polypeptide se liant à un membre de la famille IL-1R ; et
(e) un fragment du polypeptide de la SEQ ID NO :3 dont l'acide aminé N-terminal est choisi parmi les acides aminés 21-51 et dont l'acide aminé C-terminal est choisi parmi les acides aminés 188-192 et qui se lie à un membre de la famille IL-1R, le fragment ne faisant pas partie des acides aminés 24-192, 28-192 et 34-192 de la SEQ ID NO :3 ;
(f) un fragment du polypeptide de la SEQ ID NO :8 dont l'acide aminé N-terminal est choisi parmi les acides aminés 41-61 et dont l'acide aminé C-terminal est choisi parmi les acides aminés 214-218 et qui se lie à un membre de la famille IL-1R, le fragment ne faisant pas partie des acides aminés 43-218, 54-218 et 60-218 de la SEQ ID NO :8 ; et
(g) un fragment du polypeptide de la SEQ ID NO :9 dont l'acide aminé N-terminal est choisi parmi les acides aminés 21-41 et dont l'acide aminé C-terminal est choisi parmi les acides aminés 193-197 et qui se lie à un membre de la famille IL-1R, le fragment ne faisant pas partie des acides aminés 33-197 et 39-197 de la SEQ ID NO :9.

2. Polynucléotide isolé selon la revendication 1, **caractérisé en ce que** la molécule d'acide nucléique présente la séquence de la SEQ ID NO :1 ou de la SEQ ID NO :6.

3. Polynucléotide isolé selon la revendication 1, **caractérisé en ce que** l'acide aminé N-terminal du fragment du polypeptide de la SEQ ID NO :8 est l'acide aminé 52.

4. Polynucléotide isolé selon la revendication 1, **caractérisé en ce que** l'acide aminé N-terminal du fragment du polypeptide de la SEQ ID NO :9 est l'acide aminé 31.

5. Polynucléotide isolé comprenant un polypeptide choisi dans le groupe constitué de :
(a) un polypeptide comprenant la SEQ ID NO :3 ;
(b) un polypeptide comprenant la SEQ ID NO :8 ;
(c) un polypeptide comprenant la SEQ ID NO :9 ;
(d) un polypeptide qui est identique au moins à 95% par rapport à un polypeptide de a) - c), le polypeptide se liant à un membre de la famille IL-1R ; et
(e) un fragment du polypeptide de la SEQ ID NO :3 dont l'acide aminé N-terminal est choisi parmi les acides aminés 21-51 et dont l'acide aminé C-terminal est choisi parmi les acides aminés 188-192 et qui se lie à un membre de la famille IL-1R, le fragment ne faisant pas partie des acides aminés 24-192, 28-192 et 34-192 de la SEQ ID NO :3 ;
(f) un fragment du polypeptide de la SEQ ID NO :8 dont l'acide aminé N-terminal est choisi parmi les acides aminés 41-61 et dont l'acide aminé C-terminal est choisi parmi les acides aminés 214-218 et qui se lie à un membre de la famille IL-1R, le fragment ne faisant pas partie des acides aminés 43-218, 54-218 et 60-218 de la SEQ ID NO :8 ; et
(g) un fragment du polypeptide de la SEQ ID NO :9 dont l'acide aminé N-terminal est choisi parmi les acides aminés 21-41 et dont l'acide aminé C-terminal est choisi parmi les acides aminés 193-197 et qui se lie à un membre de la famille IL-1R, le fragment ne faisant pas partie des acides aminés 33-197 et 39-197 de la SEQ ID NO :9.

6. Polypeptide isolé selon la revendication 5, **caractérisé en ce que** l'acide aminé N-terminal du fragment du polypeptide de la SEQ ID NO :8 est l'acide aminé 52.

7. Polypeptide isolé selon la revendication 5, **caractérisé en ce que** l'acide aminé N-terminal du fragment du polypeptide de la SEQ ID NO :9 est l'acide aminé 31.

8. Vecteur d'expression comprenant un polynucléotide selon l'une quelconque des revendications 1 à 4.

9. Cellule hôte transformée ou transfectée par le vecteur de la revendication 8.

10. Procédé de préparation d'un polypeptide, le procédé comprenant la mise en culture d'une cellule hôte selon la revendication 9 dans des conditions promouvant l'expression du polypeptide.

11. Polypeptide oligomère comprenant un polypeptide selon la revendication 5, 6 ou 7.

12. Anticorps se liant à un polypeptide selon la revendication 5, 6, 7 ou 11.

13. Anticorps selon la revendication 12, **caractérisé en ce que** l'anticorps est un anticorps monoclonal.


### SEQUENCE LISTING

<110> Sims. John E.
   Born. Teresa L.
   Smith, Dirk E.
<120> IL-1 ZETA, IL-1 ZETA SPLICE VARIANTS AND XREC2 DNAS AND POLYPEPTIDES
<130> 03260.0088-00304
<140>
   <141>
<150> 60/112,163
   <151> 1998-12-14
<150> 60/146.675
   <151> 1999-11-10
<160> 15
<170> Patent In Ver. 2.0
<210> 1
   <211> 579
   <212> DNA
   <213> Homo sapiens
<400> 1
   atgtcaggct gtgataggag ggaaacagaa accaaaggaa agaacagctt taagaagcgc 60
   ttaagaggtc caaaggtgaa gaacttaaac ccgaagaaat tcagcattca tgaccaggat 120
   cacaaagtac tggtcctgga ctctgggaat ctcatagcag ttccagataa aaactacata 180
   cgcccagaga tcttctttgc attagcctca tccttgagct cagcctctgc ggagaaagga 240
   agtccgattc tcctgggggt ctctaaaggg gagttttgtc tctactgtga caaggataaa 300
   ggacaaagtc atccatccct tcagctgaag aaggagaaac tgatgaagct ggctgcccaa 360
   aaggaatcag cacgccggcc cttcatcttt tatagggctc aggtgggctc ctggaacatg 420
   ctggagtcgg cggctcaccc cggatggttc atctgcacct cctgcaattg taatgagcct 480
   gttggggtga cagataaatt tgagaacagg aaacacattg aattttcatt tcaaccagtt 540
   tgcaaagctg aaatgagccc cagtgaggtc agcgattag                        579
<210> 2
   <211> 2091
   <212> DNA
   <213> Homo sapiens
<400> 2
   atgaaagctc cgattccaca cttgattctc ttatacgcta cttttactca gagtttgaag 60
   gttgtgacca aaagaggctc cgccgatgga tgcactgact ggtctatcga tatcaagaaa 120
   tatcaagttt tggtgggaga gcctgttcga atcaaatgtg cactctttta tggttatatc 180
   agaacaaatt actcccttgc ccaaagtgct ggactcagtt tgatgtggta caaaagttct 240
   ggtcctggag actttgaaga gccaatagcc tttgacggaa gtagaatgag caaagaagaa 300
   gactccattt ggttccggcc aacattgcta caggacagtg gtctctacgc ctgtgtcatc 360
   agaaactcca cttactgtat gaaagtatcc atctcactga cagtgggtga aaatgacact 420
   ggactctgct ataattccaa gatgaagtat tttgaaaaag ctgaacttag caaaagcaag 480
   gaaatttcat gccgtgacat agaggatttt ctactgccaa ccagagaacc tgaaatcctt 540
   tggtacaagg aatgcaggac aaaaacatgg aggccaagta ttgtattcaa aagagatact 600
   ctgcttataa gagaagtcag agaagatgac attggaaatt atacctgtga attaaaatat 660
   ggaggctttg ttgtgagaag aactactgaa ttaactgtta cagcccctct gactgataag 720
   ccacccaagc ttttgtatcc tatggaaagt aaactgacaa ttcaggagac ccagctgggt 780
   gactctgcta atctaacctg cagagctttc tttgggtaca gcggagatgt cagtccttta 840
   atttactgga tgaaaggaga aaaatttatt gaagatctgg atgaaaatcg agtttgggaa 900
   agtgacatta gaattcttaa ggagcatctt ggggaacagg aagtttccat ctcattaatt 960
   gtggactctg tggaagaagg tgacttggga aattactcct gttatgttga aaatggaaat 1020
   ggacgtcgac acgccagcgt tctccttcat aaacgagagc taatgtacac agtggaactt 1080
   gctggaggcc ttggtgctat actcttgctg cttgtatgtt tggtgaccat ctacaagtgt 1140
   tacaagatag aaatcatgct cttctacagg aatcattttg gagctgaaga gctcgatgga 1200
   gacaataaag attatgatgc atacttatca tacaccaaag tggatcctga ccagtggaat 1260
   caagagactg gggaagaaga acgttttgcc cttgaaatcc tacctgatat gcttgaaaag 1320
   cattatggat ataagttgtt tataccagat agagatttaa tcccaactgg aacatacatt 1380
   gaagatgtgg caagatgtgt agatcaaagc aagcggctga ttattgtcat gaccccaaat 1440
   tacgtagtta gaaggggctg gagcatcttt gagctggaaa ccagacttcg aaatatgctt 1500
   gtgactggag aaattaaagt gattctaatt gaatgcagtg aactgagagg aattatgaac 1560
   taccaggagg tggaggccct gaagcacacc atcaagctcc tgacggtcat taaatggcat 1620
   ggaccaaaat gcaacaagtt gaactccaag ttctggaaac gtttacagta tgaaatgcct 1680
   tttaagagga tagaacccat tacacatgag caggctttag atgtcagtga gcaagggcct 1740
   tttggggagc tgcagactgt ctcggccatt tccatggccg cggccacctc cacagctcta 1800
   gccactgccc atccagatct ccgttctacc tttcacaaca cgtaccattc acaaatgcgt 1860
   cagaaacact actaccgaag ctatgagtac gacgtacctc ctaccggcac cctgcctctt 1920
   acctccatag gcaatcagca tacctactgt aacatcccta tgacactcat caacgggcag 1980
   cggccacaga caaaatcgag cagggagcag aatccagatg aggcccacac aaacagtgcc 2040
   atcctgccgc tgttgccaag ggagaccagt atatccagtg tgatatggtg a          2091
<210> 3
   <211> 192
   <212> PRT
   <213> Homo sapiens
<400> 3
   Met Ser Gly Cys Asp Arg Arg Glu Thr Glu Thr Lys Gly Lys Asn Ser
   1               5                   10                  15
   Phe Lys Lys Arg Leu Arg Gly Pro Lys Val Lys Asn Leu Asn Pro Lys
               20                  25                  30
   Lys Phe Ser Ile His Asp Gln Asp His Lys Val Leu Val Leu Asp Ser
           35                  40                  45
   Gly Asn Leu Ile Ala Val Pro Asp Lys Asn Tyr Ile Arg Pro Glu Ile
       50                  55                  60
   Phe Phe Ala Leu Ala Ser Ser Leu Ser Ser Ala Ser Ala Glu Lys Gly
   65                  70                  75                  80
   Ser Pro Ile Leu Leu Gly Val Ser Lys Gly Glu Phe Cys Leu Tyr Cys
                   85                  90                  95
   Asp Lys Asp Lys Gly Gln Ser His Pro Ser Leu Gln Leu Lys Lys Glu
               100                 105                 110
   Lys Leu Met Lys Leu Ala Ala Gln Lys Glu Ser Ala Arg Arg Pro Phe
           115                 120                 125
   Ile Phe Tyr Arg Ala Gln Val Gly Ser Trp Asn Met Leu Glu Ser Ala
       130                 135                 140
   Ala His Pro Gly Trp Phe Ile Cys Thr Ser Cys Asn Cys Asn Glu Pro
   145                 150                 155                 160
   Val Gly Val Thr Asp Lys Phe Glu Asn Arg Lys His Ile Glu Phe Ser
                   165                 170                 175
   Phe Gln Pro Val Cys Lys Ala Glu Met Ser Pro Ser Glu Val Ser Asp
   180 185 190
<210> 4
   <211> 696
   <212> PRT
   <213> Homo sapiens
<400> 4
<210> 5
   <211> 657
   <212> DNA
   <213> Homo sapiens
<400> 5
   atgtcctttg tgggggagaa ctcaggagtg aaaatgggct ctgaggactg ggaaaaagat 60
   gaaccccagt gctgcttaga agacccggct gtaagccccc tggaaccagg cccaagcctc 120
   cccaccatga attttgttca cacaagtcca aaggtgaaga acttaaaccc gaagaaattc 180
   agcattcatg accaggatca caaagtactg gtcctggact ctgggaatct catagcagtt 240
   ccagataaaa actacatacg cccagagatc ttctttgcat tagcctcatc cttgagctca 300
   gcctctgcgg agaaaggaag tccgattctc ctgggggtct ctaaagggga gttttgtctc 360
   tactgtgaca aggataaagg acaaagtcat ccatcccttc agctgaagaa ggagaaactg 420
   atgaagctgg ctgcccaaaa ggaatcagca cgccggccct tcatctttta tagggctcag 480
   gtgggctcct ggaacatgct ggagtcggcg gctcaccccg gatggttcat ctgcacctcc 540
   tgcaattgta atgagcctgt tggggtgaca gataaatttg agaacaggaa acacattgaa 600
   ttttcatttc aaccagtttg caaagctgaa atgagcccca gtgaggtcag cgattag 657
<210> 6
   <211> 594
   <212> DNA
   <213> Homo sapiens
<400> 6
   atgtcctttg tgggggagaa ctcaggagtg aaaatgggct ctgaggactg ggaaaaagat 60
   gaaccccagt gctgcttaga aggtccaaag gtgaagaact taaacccgaa gaaattcagc 120
   attcatgacc aggatcacaa agtactggtc ctggactctg ggaatctcat agcagttcca 180
   gataaaaact acatacgccc agagatcttc tttgcattag cctcatcctt gagctcagcc 240
   tctgcggaga aaggaagtcc gattctcctg ggggtctcta aaggggagtt ttgtctctac 300
   tgtgacaagg ataaaggaca aagtcatcca tcccttcagc tgaagaagga gaaactgatg 360
   aagctggctg cccaaaagga atcagcacgc cggcccttca tcttttatag ggctcaggtg 420
   ggctcctgga acatgctgga gtcggcggct caccccggat ggttcatctg cacctcctgc 480
   aattgtaatg agcctgttgg ggtgacagat aaatttgaga acaggaaaca cattgaattt 540
   tcatttcaac cagtttgcaa agctgaaatg agccccagtg aggtcagcga ttag 594
<210> 7
   <211> 474
   <212> DNA
   <213> Homo sapiens
<400> 7
   atgtcctttg tgggggagaa ctcaggagtg aaaatgggct ctgaggactg ggaaaaagat 60
   gaaccccagt gctgcttaga agagatcttc tttgcattag cctcatcctt gagctcagcc 120
   tctgcggaga aaggaagtcc gattctcctg ggggtctcta aaggggagtt ttgtctctac 180
   tgtgacaagg ataaaggaca aagtcatcca tcccttcagc tgaagaagga gaaactgatg 240
   aagctggctg cccaaaagga atcagcacgc cggcccttca tcttttatag ggctcaggtg 300
   ggctcctgga acatgctgga gtcggcggct caccccggat ggttcatctg cacctcctgc 360
   aattgtaatg agcctgttgg ggtgacagat aaatttgaga acaggaaaca cattgaattt 420
   tcatttcaac cagtttgcaa agctgaaatg agccccagtg aggtcagcga ttag 474
<210> 8
   <211> 218
   <212> PRT
   <213> Homo sapiens
<400> 8
   Met Ser Phe Val Gly Glu Asn Ser Gly Val Lys Met Gly Ser Glu Asp
   1 5 10 15
   Trp Glu Lys Asp Glu Pro Gln Cys Cys Leu Glu Asp Pro Ala Val Ser
   20 25 30
   Pro Leu Glu Pro Gly Pro Ser Leu Pro Thr Met Asn Phe Val His Thr
   35 40 45
   Ser Pro Lys Val Lys Asn Leu Asn Pro Lys Lys Phe Ser Ile His Asp
   50 55 60
   Gln Asp His Lys Val Leu Val Leu Asp Ser Gly Asn Leu Ile Ala Val
   65 70 75 80
   Pro Asp Lys Asn Tyr Ile Arg Pro Glu Ile Phe Phe Ala Leu Ala Ser
   85 90 95
   Ser Leu Ser Ser Ala Ser Ala Glu Lys Gly Ser Pro Ile Leu Leu Gly
   100 105 110
   Val Ser Lys Gly Glu Phe Cys Leu Tyr Cys Asp Lys Asp Lys Gly Gln
   115 120 125
   Ser His Pro Ser Leu Gln Leu Lys Lys Glu Lys Leu Met Lys Leu Ala
   130 135 140
   Ala Gln Lys Glu Ser Ala Arg Arg Pro Phe Ile Phe Tyr Arg Ala Gln
   145 150 155 160
   Val Gly Ser Trp Asn Met Leu Glu Ser Ala Ala His Pro Gly Trp Phe
   165 170 175
   Ile Cys Thr Ser Cys Asn Cys Asn Glu Pro Val Gly Val Thr Asp Lys
   180 185 190
   Phe Glu Asn Arg Lys His Ile Glu Phe Ser Phe Gln Pro Val Cys Lys
   195 200 205
   Ala Glu Met Ser Pro Ser Glu Val Ser Asp
   210 215
<210> 9
   <211> 197
   <212> PRT
   <213> Homo sapiens
<400> 9
   Met Ser Phe Val Gly Glu Asn Ser Gly Val Lys Met Gly Ser Glu Asp
   1 5 10 15
   Trp Glu Lys Asp Glu Pro Gln Cys Cys Leu Glu Gly Pro Lys Val Lys
   20 25 30
   Asn Leu Asn Pro Lys Lys Phe Ser Ile His Asp Gln Asp His Lys Val
   35 40 45
   Leu Val Leu Asp Ser Gly Asn Leu Ile Ala Val Pro Asp Lys Asn Tyr
   50 55 60
   Ile Arg Pro Glu Ile Phe Phe Ala Leu Ala Ser Ser Leu Ser Ser Ala
   65 70 75 80
   Ser Ala Glu Lys Gly Ser Pro Ile Leu Leu Gly Val Ser Lys Gly Glu
   85 90 95
   Phe Cys Leu Tyr Cys Asp Lys Asp Lys Gly Gln Ser His Pro Ser Leu
   100 105 110
   Gln Leu Lys Lys Glu Lys Leu Met Lys Leu Ala Ala Gln Lys Glu Ser
   115 120 125
   Ala Arg Arg Pro Phe Ile Phe Tyr Arg Ala Gln Val Gly Ser Trp Asn
   130 135 140
   Met Leu Glu Ser Ala Ala His Pro Gly Trp Phe Ile Cys Thr Ser Cys
   145 150 155 160
   Asn Cys Asn Glu Pro Val Gly Val Thr Asp Lys Phe Glu Asn Arg Lys
   165 170 175
   His Ile Glu Phe Ser Phe Gln Pro Val Cys Lys Ala Glu Met Ser Pro
   180 185 190
   Ser Glu Val Ser Asp
   195
<210> 10
   <211> 157
   <212> PRT
   <213> Homo sapiens
<400> 10
   Met Ser Phe Val Gly Glu Asn Ser Gly Val Lys Met Gly Ser Glu Asp
   1 5 10 15
   Trp Glu Lys Asp Glu Pro Gln Cys Cys Leu Glu Glu Ile Phe Phe Ala
   20 25 30
   Leu Ala Ser Ser Leu Ser Ser Ala Ser Ala Glu Lys Gly Ser Pro Ile
   35 40 45
   Leu Leu Gly Val Ser Lys Gly Glu Phe Cys Leu Tyr Cys Asp Lys Asp
   50 55 60
   Lys Gly Gln Ser His Pro Ser Leu Gln Leu Lys Lys Glu Lys Leu Met
   65 70 75 80
   Lys Leu Ala Ala Gln Lys Glu Ser Ala Arg Arg Pro Phe Ile Phe Tyr
   85 90 95
   Arg Ala Gln Val Gly Ser Trp Asn Met Leu Glu Ser Ala Ala His Pro
   100 105 110
   Gly Trp Phe Ile Cys Thr Ser Cys Asn Cys Asn Glu Pro Val Gly Val
   115 120 125
   Thr Asp Lys Phe Glu Asn Arg Lys His Ile Glu Phe Ser Phe Gln Pro
   130 135 140
   Val Cys Lys Ala Glu Met Ser Pro Ser Glu Val Ser Asp
   145 150 155
<210> 11
   <211> 8
   <212> PRT
   <213> Artificial Sequence
<220>
   <223> Description of Artificial Sequence: antigenic peptide used in fusion proteins
<400> 11
   Asp Tyr Lys Asp Asp Asp Asp Lys
   1 5
<210> 12
   <211> 27
   <212> PRT
   <213> Artificial Sequence
<220>
   <223> Description of Artificial Sequence: leucine zipper polypeptide
<400> 12
   Pro Asp Val Ala Ser Leu Arg Gln Gln Val Glu Ala Leu Gln Gly Gln
   1 5 10 15
   Val Gln His Leu Gln Ala Ala Phe Ser Gln Tyr
   20 25
<210> 13
   <211> 33
   <212> PRT
   <213> Artificial Sequence
<220>
   <223> Description of Artificial Sequence: leucine zipper polypeptide
<400> 13
   Arg Met Lys Gln Ile Glu Asp Lys Ile Glu Glu Ile Leu Ser Lys Ile
   1 5 10 15
   Tyr His Ile Glu Asn Glu Ile Ala Arg Ile Lys Lys Leu Ile Gly Glu
   20 25 30
   Arg
<210> 14
   <211> 8
   <212> PRT
   <213> Artificial Sequence
<220>
   <223> Description of Artificial Sequence: polymorphic sequence from exon 2 of Tango 77
<400> 14
   Pro Ala Gly Ser Pro Leu Glu Pro
   1 5
<210> 15
   <211> 8
   <212> PRT
   <213> Artificial Sequence
<220>
   <223> Description of Artificial Sequence: polymorphic sequence from exon 2 of Tango 77
<400> 15
   Pro Ala Val Ser Pro Leu Glu Pro
   1 5